# EUROPEAN PATENT SPECIFICATION

(11) **EP 3 883 393 B1**
(45) Date of publication and mention of the grant of the patent: **07.09.2022**
(21) Application number: 20730042.7
(22) Date of filing: 04.06.2020
(51) Int. Cl.: A23L 33/10, A23L 33/12, A23L 33/21, A23L 33/00, A61K 31/19, A61K 31/702, A23C 9/20, A61P 3/02

(54) **NUTRITIONAL COMPOSITION COMPRISING 2'FUCOSYLLACTOSE AND DIETARY BUTYRATE**
NÄHRSTOFFZUSAMMENSETZUNG ENTHALTEND 2'FUCOSYLLACTOSE UND BUTYRAT
COMPOSITION NUTRITIONNELLE COMPRENANT DU 2'-FUCOSYLLACTOSE ET DU BUTYRATE ALIMENTAIRE

(30) Priority: 04.06.2019 EP 19178299
(43) Date of publication of application: 29.09.2021
(73) Proprietor: N.V. Nutricia, 2712 HM Zoetermeer (NL)
(72) Inventor: POTAPPEL - van 't LAND, Belinda, 3584 CT Utrecht (NL); RENES, Ingrid Brunhilde, 3584 CT Utrecht (NL); WIERTSEMA, Selma Paulien, 3584 CT Utrecht (NL); THOMASSEN, Gabriël, 3584 CT Utrecht (NL); OVERBEEK, Saskia Adriana, 3584 CT Utrecht (NL); BEN AMOR, Kaouther, 3584 CT Utrecht (NL); BRABER, Saskia, 3584 CT Utrecht (NL)
(74) Representative: Nederlandsch Octrooibureau
(86) International application number: PCT/EP2020/065547
(87) International publication number: WO 2020/245311

(56) References cited:
- CN-A- 107 736 614
- US-A1- 2007 104 700
- US-A1- 2012 129 935
- US-A1- 2015 064 220
- US-A1- 2017 157 242
- US-A1- 2019 029 306
- KOBATA A: "Structures and application of oligosaccharides in human milk", PROCEEDINGS OF THE JAPAN ACADEMY. SERIES B, PHYSICAL ANDBIOLOGICAL SCIENCES, TOKYO, JP, vol. 86, no. 7, 1 July 2010 (2010-07-01), pages 731-747, XP002612998, ISSN: 0386-2208, DOI: 10.2183/PJAB.86.731

## Description

### FIELD OF THE INVENTION

The present invention relates to the field of infant and young child formula and the improvement of the intestinal health.

### BACKGROUND OF THE INVENTION

Human milk is the preferred food for infants. Human milk provides several bioactive factors that benefit the relatively immature immune system and the intestinal health of neonates early in life. Human milk fed infants have a lower incidence of infections than formula fed infants. Many components in human milk, including immunoglobulins (such as slgA), interleukin (IL)-1, IL-6, IL-8, IL-10, interferon-γ (IFN-γ), immunocompetent cells, transforming growth factor-β (TGF-β), lactoferrin, nucleotides and human milk oligosaccharides (HMOs) are thought to play a role in protection against infection with pathogens. Additionally intestinal maturation and development of the microbiota in human milk fed infants is considered optimal.

However, it is not always possible or desirable to breastfeed an infant. In such cases infant formulae or follow-on formulae are a good alternative. These formulae should have an optimal composition in order to mimic the beneficial effects of human milk as close as possible.

WO 2005/122790 discloses a method for stimulating barrier integrity by administering a composition comprising eicosapentaenoic acid (EPA), docosahexaenoic acid (DHA) and arachidonic acid (ARA), and at least two distinct oligosaccharides. The oligosaccharides act indirectly by being fermented to short chain fatty acids (SCFA) by the intestinal microbiota.

WO 2016/013935 discloses the use of a non-digestible oligosaccharide in the manufacture of a composition for providing nutrition to an infant suffering from an increased risk of food allergy. The infant is preferably at increased risk of trichothecene mycotoxin exposure, for instance by eating a lot of cereals. In the examples VivinalGOS is the source of galacto-oligosaccharides.

WO 2006/115412 relates to a liquid nutritional composition for improving intestinal barrier function and for preventing allergy. The composition comprises lipids with short chain fatty acyl chains and non-digestible, fermentable saccharides.

WO 2004/112509 discloses a composition for inducing a pattern of intestinal barrier maturation similar to that observed with breast-feeding. The composition helps to improve intestinal barrier maturation, e.g. during neonatal stress. It is disclosed that maternal separation in rats increases the intestinal permeability and that a blend containing LC-PUFA, *Lactobacillus paracasei* and non-digestible oligosaccharides can restore the intestinal permeability to normal levels.

US 2019029306 discloses the use of compositions comprising fucosylated oligosaccharides such as 2'-fucosyllactose, in the prevention and treatment of skin conditions, such as atopic dermatitis, in infants and young children.

Still there is a need to improve infant formulae and compositions for young children to come closer to human milk in structure and function.

### SUMMARY OF INVENTION

The inventors have found that a combination of the nutritional ingredients 2'-FL and dietary butyric acid has a beneficial effect on the intestinal barrier function. Also it has been found that the response of the immune system and microbiota are different when both 2'-FL and dietary butyric are present compared to when only one of these ingredients is present. The mixture of 2'-FL and dietary butyric acid has been shown to have a beneficial effect on alkaline phosphatase expression which is indicative for an improved intestinal barrier function maturation and an improved defense against intestinal pathogenic bacteria. These effects were further improved by the presence of 3'-GL. Hence a nutritional composition comprising both dietary butyric acid and 2'-FL and preferably additionally 3'-GL, will have improved health effects for infants and young children.

### DETAILED DESCRIPTON:

The present invention relates to a nutritional composition, which is not human milk, for infants or young children comprising:
a. 2'fucosyllactose, and
b. dietary butyrate.

In a preferred embodiment the nutritional composition further comprises 3'galactosyllactose.

In another or further preferred embodiment the nutritional composition is at least partly fermented by lactic acid producing bacteria and comprises 0.1 to 1.5 wt.% of the sum of lactic acid and lactate based on dry weight of the nutritional composition, and wherein at least 90 wt.% of the sum of lactic acid and lactate is L-lactic acid and L-lactate.

The disclosure further relates to said nutritional composition for use as a medicament, preferably for the treatment, prevention and/or alleviation of a disease and/or illness. The nutritional composition is for use in treatment or prevention of infections, in particular intestinal infections, and/or for use in inducing oral tolerance to allergens.

### Definitions

In the context of the present invention the term "prevention" means "reducing the risk of (occurrence)" or "reducing the severity of". The term "prevention of a certain condition" also includes "treatment of a person at (increased) risk of said condition".

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### 2'-Fucosyllactose

The nutritional composition of the present invention comprises 2'-fucosyllactose (2-'FL). 2'-FL was found to improve the intestinal barrier function. Also 2'-FL was found to improve the immune system. Fucosyllactose (FL) is a non-digestible oligosaccharide present in human milk. It is not present in bovine milk. It consists of three monose units, fucose, galactose and glucose linked together. Lactose is a galactose unit linked to a glucose unit via a beta 1,4 linkage. A fucose unit is linked to a galactose unit of a lactose molecule via an alpha 1,2 linkage (2'-fucosyllactose, 2'-FL) or via an alpha-1,3 linkage to the glucose unit of a lactose (3-Fucosyllactose, 3-FL).

2'-FL, preferably α-L-Fuc-(1→2)-β-D-Gal-(1→4)-D-Glc, is commercially available, for instance from Sigma-Aldrich. Alternatively, it can be isolated from human milk, for example as described in Andersson & Donald, 1981, J Chromatogr. 211:170-1744, or produced by genetically modified micro-organisms, for example as described in Albermann et al, 2001, Carbohydrate Res. 334:97-103.

Preferably, a nutritional composition according to the invention comprises 10 mg to 1 g 2'-FL per 100 ml, more preferably 20 mg to 0.5 g, even more preferably 40 mg to 0.2 g 2'-FL per 100 ml. Based on dry weight, the present nutritional composition preferably comprises 0.075 wt.% to 7.5 wt.% 2'-FL, more preferably 0.15 wt.% to 3.75 wt.% 2'-FL, even more preferably 0.3 wt.% to 1.5 wt.% 2'-FL. Based on energy, the present nutritional composition preferably comprises 0.015 to 1.5 g 2'-FL per 100kcal, more preferably 0.03 to 0.075 g 2'-FL per 100 kcal, even more preferably 0.06 to 0.3 g 2'-FL per 100 kcal. A lower amount of fucosyllactose will be less effective in stimulating the immune system or improving the intestinal barrier function, whereas a too high amount will result in unnecessary high costs of the product.

### Dietary butyrate

The present nutritional composition contains dietary butyrate. Butyrate was found to improve the intestinal barrier function. The nutritional composition preferably comprises between 0.3 and 5 wt.% butyric acid based on based on weight of total fatty acyl chains, preferably between 0.6 and 5 wt.%, even more preferably between 1 and 5 wt.%. The present nutritional composition preferably contains tributyrin (i.e. triglyceride with 3 butyric acid chains attached to the glycerol backbone via ester bonds). Preferably the nutritional composition contains 0.075 to 1.3 wt.% butyrate based on dry weight of the composition, preferably between 0.15 and 1.3 wt.% and more preferably between 0.25 and 1.3 wt.%. Alternatively the nutritional composition comprises 0.015 to 0.25 g butyrate per 100 kcal, preferably 0.03 to 0.25 g butyrate per 100 kcal, and more preferably 0.05 to 0.25 g butyrate per 100 kcal. When the nutritional composition is a liquid, the composition preferably contains 0.01 to 0.175 g butyrate per 100 ml, more preferably 0.02 to 0.175 g butyrate per 100 ml, and more preferably 0.035 to 0.175 g butyrate per 100 ml. It is known that human milk contains very low levels of butyrate, in particular < 0.1 wt.% based on total fatty acids.

The dietary butyrate can be supplied by any suitable source known in the art. Non-limiting sources of dietary butyrate includes animal source fats and derived products, such as but not limited to milk, milk fat, butter fat, butter oil, butter, buttermilk, butter serum, cream; microbial fermentation derived products, such as but not limited to yogurt and fermented buttermilk; and plant source derived seed oil products, such as pineapple and/or pineapple oil, apricot and/or apricot oil, barley, oats, brown rice, bran, green beans, legumes, leafy greens, apples, kiwi, oranges. In some embodiments, the dietary butyrate is synthetically produced. The preferred source of dietary butyrate is milk fat from ruminants, preferably bovine milk fat.

In embodiments where the dietary butyrate is synthetically produced, the chemical structure of the dietary butyrate may be modified as necessary. Further, the dietary butyrate produced synthetically can be purified by any means known in the art to produce a purified dietary butyrate additive that can be incorporated into the nutritional compositions disclosed herein. The dietary butyrate may be provided by dairy lipids and/or triglyceride bound forms of butyrate.

In some embodiments, the dietary butyrate may comprise butyrate salts, for example, sodium butyrate, potassium butyrate, calcium butyrate, magnesium butyrate, and combinations thereof. In certain embodiments, dietary butyrate comprises a suitable butyrate salt that has been coated with one or more fats or lipids. In certain embodiments wherein the dietary butyrate comprises a fat-coated butyrate salt, the nutritional composition may be a dry-powdered composition into which the dietary butyrate is incorporated. Preferably the dietary butyrate is supplied as part of a triglyceride. This is advantageous because butyrate is volatile (and malodorous) when provided in free or salt form. In triglyceride form the butyrate will be released in and after the stomach due to the action of lipases.

In a preferred embodiment, the weight ratio of 2'-FL to dietary butyrate is in the range of 10:1 to 1:10, preferably 5:1 to 1:5, more preferably 3:1 to 1:3.

### 3'galactosyllactose

The nutritional composition of the present invention preferably comprises 3'-galactosyllactose. Preferably the 3'-galactosyllactose is the trisaccharide Gal-(beta 1,3)-Gal-(beta 1,4)-Glc. In the context of the invention, all mentions of 3-'GL refers to beta1,3'-galactosyllactose or beta3'-GL, unless specifically indicated that this is not the case. This trisaccharide can be administered in a suitable matrix, or in a nutritional composition. The trisaccharide may for example be part of a mixture of galacto-oligosaccharides (GOS), preferably beta-galacto-oligosaccharides (betaGOS). Beta3'-GL was found to improve the intestinal barrier function.

The nutritional composition according to the present invention preferably comprises 0.07 to 3.75 wt.% Gal (beta 1-3) - Gal (beta 1-4) - Glc, based on dry weight of the nutritional composition. In a preferred embodiment, the nutritional composition comprises 0.07 to 0.375 wt.% Gal (beta 1-3) - Gal (beta 1-4) - Glc, based on dry weight of the nutritional composition. In another preferred embodiment, the nutritional composition comprises 1.125 to 1.725 wt.% Gal (beta 1-3) - Gal (beta 1-4) - Glc, based on dry weight of the nutritional composition.

The nutritional composition according to the present invention preferably comprises 15 to 750 mg Gal (beta 1-3) - Gal (beta 1-4) - Glc, per 100 kcal of the nutritional composition. In a preferred embodiment, the nutritional composition comprises 15 to 75 mg Gal (beta 1-3) - Gal (beta 1-4) - Glc, per 100 kcal of the nutritional composition. In another preferred embodiment, the nutritional composition comprises 225 to 375 mg Gal (beta 1-3) - Gal (beta 1-4) - Glc, per 100 kcal of the nutritional composition.

The nutritional composition according to the present invention preferably comprises 10 to 500 mg Gal (beta 1-3) - Gal (beta 1-4) - Glc, per 100 ml of the nutritional composition. In a preferred embodiment, the nutritional composition comprises 10 to 50 mg Gal (beta 1-3) - Gal (beta 1-4) - Glc, per 100 ml of the nutritional composition. In another preferred embodiment, the nutritional composition comprises 150 to 250 mg Gal (beta 1-3) - Gal (beta 1-4) - Glc, per 100 ml of the nutritional composition. It is known that human milk contains low levels of 3'-GL, in particular on average not exceeding 5 mg/100 ml. The combination of 2'-FL, butyrate and 3'-GL will have a further improved effect on health, in particular on improving the intestinal barrier function, on improving the immune system, on improving the intestinal microbiota and/or on the treatment or prevention of infections, in particular intestinal infections.

In a preferred embodiment, the weight ratio of 2'-FL to 3'-GL is in the range of 10:1 to 1:10, preferably 5:1 to 1:5, more preferably 3:1 to 1:3.

### Other oligosaccharides

If present, the beta1,3'-galactosyllactose may be part of a mixture of galacto-oligosaccharides (GOS), preferably beta-galacto-oligosaccharides (BGOS). It is advantageous to add GOS to the present nutritional composition, in addition to beta1,3'-galactosyllactose (beta3'-GL) specifically. A mixture of GOS with different sizes and linkages will have an increased beneficial effect on the microbiota and an improved production of short chain fatty acids, which in its turn have a further improving effect on the immune system and/or on treatment or prevention of infections, in particular intestinal infections. The presence of GOS other than beta3'-GL will in particular have an additional effect on the intestinal barrier function in the large intestine and end of the small intestine, whereas the beta3'-GL will be also - and mostly - effective in the small intestine. The addition of 3'-GL and GOS therefore will have a further improved effect on health, in particular on improving the intestinal barrier function, on improving the immune system, on improving the intestinal microbiota and/or on the treatment or prevention of infections, in particular intestinal infections.

In the context of the invention, a suitable way to form GOS is to treat lactose with beta-galactosidases. Dependent on the specificity of the enzyme used, a galactose unit is hydrolysed from lactose and coupled to another lactose unit via a beta-linkage to form a trisaccharide. A galactose unit may also be coupled to another single galactose unit to form a disaccharide. Subsequent galactose units are coupled to form oligosaccharides. The majority of such formed oligosaccharides have a degree of polymerization (DP) of 7 or lower. Depending on the enzyme these linkages between the galactose residues can be predominantly beta1,4', beta1,6' or beta1,3'.

A suitable way to prepare beta1,6' and/or beta1,4' GOS is by using the beta-galactosidase from *Bacillus circulans.* A commercially available source of BGOS is Vivinal-GOS from FrieslandCampina Domo (Amersfoort, The Netherlands). Vivinal-GOS comprises BGOS mainly with DP2-8 (peak at DP3) and mainly with beta1,4' and beta1,6' linkages, with beta1,4' linkages being more predominant. Beta1,4'- and beta1,6'-galactosyl-lactose can be enriched or purified from these GOS mixtures as known in the art, for example by size exclusion chromatography. Other commercially available source of BGOS with predominantly beta1,4' and/or beta 1,6' linkages are Oligomate 55 and 50 from Yakult, and Cup Oligo form Nissin Sugar. Alternatively beta1,4'- and beta1,6'-galactosyllactose are commercially available as single components (Carbosynth).

A suitable way to produce beta1,3' GOS, is by using a beta-galactosidase from S. *thermophilus.* Particularly suitable is the use of beta-galactosidase from strain CNCM I-1470 and/or CNCM I-1620 in a process as disclosed in example 4 of FR2723960 or example 6 of EP0778885. S. *thermophilus* CNCM I-1620 was deposited under the Budapest Treaty on 23 August 1995 at Collection Nationale de Cultures de Microorganisms van Institute Pasteur, Paris, France by Compagnie Gervais Danone. Strain S. *thermophilus* CNCM I-1620 is also referred to as strain S. *thermophilus* ST065. S. *thermophilus* CNCM I-1470 was deposited under the Budapest Treaty on 25 August 1994 at Collection Nationale de Cultures de Microorganisms van Institute Pasteur, Paris, France by Compagnie Gervais Danone. The composition of this GOS is also described in more detail in LeForestier et. al., 2009 Eur J Nutr, 48:457-464. Both strains have also been published in WO 96/06924. Another commercially available GOS rich in beta1,3 and beta1,6 galacto-oligosaccharides is Bimuno from Clasado, or Purimune from GTC Nutrition. Beta1,6'- and beta1,3'-galactosyl-lactose can be enriched or purified from these GOS mixtures as known in the art, for example by size exclusion chromatography. Alternatively, pure beta1,3'-galactosyl-lactose is commercially available (Carbosynth).

The GOS, including BGOS, are non-digestible. Human digestive enzymes (including human lactase) are not able to hydrolyse GOS. GOS when consumed therefore reaches the large intestine intact and is available for fermentation by the intestinal microbiota.

Preferably the nutritional composition comprises at least 250 mg GOS per 100 ml, more preferably at least 400 even more preferably at least 600 mg per 100 ml. Preferably the nutritional composition does not comprise more than 2500 mg of GOS per 100 ml, preferably not more than 1500 mg, more preferably not more than 1000 mg. More preferably, the nutritional composition according to the present invention comprises GOS in an amount of 250 to 2500 mg/100 ml, even more preferably in an amount of 400 to 1500 mg/100ml, even more preferably in an amount of 600 to 1000 mg/100 ml.

Preferably the nutritional composition comprises at least 1.75 wt.% of GOS based on dry weight of the total composition, more preferably at least 2.8 wt.%, even more preferably at least 4.2 wt.%, all based on dry weight of the total composition. Preferably the nutritional composition does not comprise more than 17.5 wt.% of GOS based on dry weight of the total composition, more preferably not more than 10.5 wt.%, even more preferably not more than 7 wt.%. The nutritional composition according to the present invention preferably comprises GOS in an amount of 1.75 to 17.5 wt.%, more preferably in an amount of 2.8 to 10.5 wt.%, most preferably in an amount of 4.2 to 7 wt.%, all based on dry weight of the total composition.

Preferably the nutritional composition according to the present invention comprises at least 0.35 g GOS per 100 kcal, more preferably at least 0.6 g, even more preferably at least 0.8 g per 100 kcal. Preferably the nutritional composition does not comprise more than 3.7 g of GOS per 100 kcal, preferably not more than 2.5 g per 100 kcal, more preferably not more than 1.5 g per 100 kcal. More preferably, the nutritional composition according to the present invention comprises GOS in an amount of 0.35 to 3.7 g per 100 kcal, even more preferably in an amount of 0.6 to 2.5 g per 100ml, even more preferably in an amount of 0.8 to 1.5 g per 100 ml.

Lower amounts result in a less effective composition, whereas the presence of higher amounts of GOS may result in side-effects such as osmotic disturbances, abdominal pain, bloating, gas formation and/or flatulence.

The total amount of GOS as defined for the present nutritional composition is including the amount of beta1,3'-galactosyllactose.

In a preferred embodiment, the nutritional composition comprises 0.25 to 2.5 g galacto-oligosaccharides per 100 ml, wherein 10 mg to 500 mg per 100 ml of the galacto-oligosaccharides is Gal (beta 1-3) - Gal (beta 1-4) - Glc. In another preferred embodiment, the nutritional composition comprises 0.25 to 2.5 g galacto-oligosaccharides per 100 ml, wherein the amount of Gal (beta 1-3) - Gal (beta 1-4) - Glc is more than 20 wt.% based on total galacto-oligosaccharides. In another preferred embodiment, the nutritional composition comprises 0.25 to 2.5 g g galacto-oligosaccharides per 100 ml, wherein the amount of Gal (beta 1-3) - Gal (beta 1-4) - Glc is between 10-500 mg per 100 ml. In another preferred embodiment, the nutritional composition comprises 0.25 to 2.5 g galacto-oligosaccharides per 100 ml, wherein the amount Gal (beta 1-3) - Gal (beta 1-4) - Glc is more than 20 wt.% based on total galacto-oligosaccharides and wherein the amount of Gal (beta 1-3) - Gal (beta 1-4) - Glc is between 150 mg and 250 mg per 100 ml.

In another preferred embodiment, the nutritional composition comprises 0.25 to 2.5 g galacto-oligosaccharides per 100 ml, wherein the amount Gal (beta 1-3) - Gal (beta 1-4) - Glc is is between 10 mg and 50 mg per 100 ml.

The amount of beta1,3'-galactosyl-lactose in this GOS preparation is preferably in the range of 60-65wt.%, based on total galacto-oligosaccharides (excluding lactose, galactose and glucose). Other preferred sources of beta1,3'-galactosyl-lactose include Bimuno (Clasado) or Purimune (GTC Nutrition). Preferably - as further explained below - the nutritional composition according to the present invention also comprises fructo-oligosaccharides (FOS).

### Fermented composition

The present nutritional composition is preferably at least party fermented. A partly fermented nutritional composition comprises at least for a part a composition that was fermented by lactic acid producing bacteria. It was shown that a partly fermented formula has a protective effect on maintaining the intestinal permeability when exposed to physical or psychological stress.

The fermentation preferably takes place during the production process of the nutritional composition. Preferably, the nutritional composition does not contain significant amounts of viable bacteria in the final product, and this can be achieved by heat inactivation after fermentation or inactivation by other means. Preferably the fermented composition is a milk-derived product, which is a milk substrate that is fermented by lactic acid producing bacteria, wherein the milk substrate comprises at least one selected from the group consisting of milk, whey, whey protein, whey protein hydrolysate, casein, casein hydrolysate or mixtures thereof. Suitably, nutritional compositions comprising fermented compositions and non-digestible oligosaccharide and their way of producing them are described in WO 2009/151330, WO 2009/151331 and WO 2013/187764.

The fermented composition preferably comprises bacterial cell fragments like glycoproteins, glycolipids, peptidoglycan, lipoteichoic acid (LTA), lipoproteins, nucleotides, and/or capsular polysaccharides. It is of advantage to use the fermented composition comprising inactivated bacteria and/or cell fragments directly as a part of the final nutritional product, since this will result in a higher concentration of bacterial cell fragments. When commercial preparations of lactic acid producing bacteria are used, these are usually washed and material is separated from the aqueous growth medium comprising the bacterial cell fragments, thereby reducing or eliminating the presence of bacterial cell fragments. Furthermore, upon fermentation and/or other interactions of lactic acid producing bacteria with the milk substrate, additional bio-active compounds can be formed, such as short chain fatty acids, bioactive peptides and/or oligosaccharides, and other metabolites, which may also result in an intestinal microbiota-function more similar to the intestinal microbiota-function of breastfed infants. Such bioactive compounds that are produced during fermentation by lactic acid producing bacteria may also be referred to as post-biotics. A composition comprising such post-biotics is thought to be advantageously closer to breast milk, as breast milk is not a clean synthetic formula, but contains metabolites, bacterial cells, cell fragments and the like. Therefore the fermented composition, in particular fermented milk-derived product, is believed to have an improved effect compared to non-fermented milk-derived product without or with merely added lactic acid producing bacteria on the prevention of precocious maturation of the intestine in an infant, and inducing, in an infant, an intestinal maturation pattern which is more similar to the intestinal maturation pattern observed in human milk fed infants.

Preferably the final nutritional composition comprises 5 to 97.5 wt.% of the fermented composition based on dry weight, more preferably 10 to 90 wt.%, more preferably 20 to 80 wt.%, even more preferably 25 to 60 wt.%. As a way to specify that the final nutritional composition comprises at least partly a fermented composition, and to specify the extent of fermentation, the level of the sum of lactic acid and lactate in the final nutritional composition can be taken, as this is the metabolic end product produced by the lactic acid producing bacteria upon fermentation. The present final nutritional composition preferably comprises 0.1 to 1.5 wt.% of the sum of lactic acid and lactate based on dry weight of the composition, more preferably 0.15 to 1.0 wt.%, even more preferably 0.2 to 0.5 wt.%. Alternatively the nutritional composition comprises 0.02 to 0.3 g of the sum of lactic acid and lactate per 100 kcal, preferably 0.03 to 0.2 of the sum of lactic acid and lactate per 100 kcal, preferably 0.04 to 0.1 of the sum of lactic acid and lactate per 100 kcal. Alternatively, when the composition is a liquid, the sum of lactic acid and lactate is 0.0125 to 0.2 g per 100ml, preferably 0.02 to 0.125 g per 100 ml, preferably 0.03 to 0.07 g per 100 ml.

Preferably at least 50 wt.%, even more preferably at least 90 wt.%, of the sum of lactic acid and lactate is in the form of the L(+)-isomer. Thus in one embodiment the sum of L(+)-lactic acid and L(+)-lactate is more than 50 wt.%, more preferably more than 90 wt.%, based on the sum of total lactic acid and lactate. Herein L(+)-lactate and L(+)-lactic acid is also referred to as L-lactate and L-lactic acid.

The combination of 2'-FL, butyrate, optional 3'-GL and partly fermented formula will have a further improved effect on health, in particular on improving the intestinal barrier function, on improving the immune system, on improving the intestinal microbiota and/or on the treatment or prevention of infections, in particular intestinal infections.

### LCPUFA

The present nutritional composition preferably comprises long chain poly-unsaturated fatty acids (LC-PUFA). LC-PUFA are fatty acids or fatty acyl chains with a length of 20 to 24 carbon atoms, preferably 20 or 22 carbon atoms, comprising two or more unsaturated bonds. Preferably the nutritional composition comprises at least one, preferably two, more preferably three LC-PUFA selected from docosahexaenoic acid (DHA), eicosapentaenoic acid (EPA) and arachidonic acid (ARA). These LC-PUFA were found to improve the intestinal barrier function and may therefore be particularly advantageously combined with 2-'FL, butyrate and optional 3'-GL in order to further improve the intestinal barrier. This combination has unexpected advantageous effects and preferably works synergistically. Preferably the nutritional composition comprises an elevated amount of such LC-PUFA. Current infant formula, in the case they comprise these LC-PUFA, typically have an amount of the sum of DHA, ARA and EPA of 0.4 to 0.9 wt.% based on total fatty acids. In the nutritional composition according to the present invention, preferably the amount of these LC-PUFA is above 1 wt.%, preferably above 1.1 wt.%, based on total fatty acids. Preferably the amount of these LC-PUFA is not more than 15 wt.%, preferably not more than 5 wt.%, based on total fatty acids, preferably not more than 2.5 wt, based on total fatty acids. It is further preferred that the amount of these LC-PUFA is in the range of 1-15 wt.%, preferably 1.1-5 wt.%, more preferably 1.5-2.5 wt.% based on total fatty acids. This is considered most optimal range to be used in infant formula for improvement of intestinal barrier function. Preferably the amount of DHA is at least 0.4, preferably at least 0.5 wt.%, based on total fatty acids. Preferably the amount of DHA is not more than 1 wt.%, preferably not more than 0.7 wt.%, based on total fatty acids. Preferably the nutritional composition comprises an amount of DHA of at least 0.5 wt.%, preferably at least 0.7 wt.%, more preferably at least 1 wt.%, based on total fatty acids. Preferably the nutritional composition comprises an amount of DHA of 0.4 to 1 wt.%, more preferably 0.5 to 0.7 wt.%. Preferably the nutritional composition comprises an amount of EPA of at least 0.09 wt.%, preferably at least 0.1 wt.%, based on total fatty acids, and preferably not more than 0.4 wt.%, more preferably not more than 0.1 wt.%. Preferably the nutritional composition comprises an amount of EPA of 0.09 to 0.4 wt.%, more preferably 0.1 to 0.2 wt.%.

Preferably the nutritional composition comprises an amount of ARA of at least 0.25 wt.% based on total fatty acids, more preferably at least 0.5 wt.% and preferably not more than 1 wt.%. Preferably the nutritional composition comprises an amount of ARA of 0.4 to 1 wt.%, more preferably 0.5 to 0.7 wt.%. Preferably the nutritional composition comprises DHA in amount of 0.4 to 1.0 wt.% based on total fatty acids, and EPA in an amount of 0.09 to 0.4 wt.% based on total fatty acids. More preferably, the nutritional composition comprises DHA in amount of 0.5 to 0.7 wt.% based on total fatty acids, and EPA in an amount of 0.1 to 0.2 wt.% based on total fatty acids. It is particularly preferred that the nutritional composition comprises DHA in amount of more than 0.5 wt.% based on total fatty acids, and EPA in an amount of more than 0.1 wt.% based on total fatty acids. Preferably the nutritional composition comprises DHA, EPA, and ARA in amount of 0.4 to 1.0 wt.%, of 0.09 to 0.4 wt.%, and of 0.25 to 1.0 wt % based on total fatty acids, respectively. More preferably the nutritional composition comprises DHA, EPA, and ARA in amount of 0.5 to 0.7 wt.%, of 0.1 to 0.2 wt.%, and of 0.5 to 0.7 wt % based on total fatty acids, respectively.

Preferably the nutritional composition comprises DHA in amount of 20 to 50 mg/100 kcal and EPA in an amount of 4.3 to 10.8 mg/100 kcal. More preferably the nutritional composition comprises DHA in an amount of 25 to 33.5 mg/100 kcal and EPA in an amount of 5.4 to 7.2 mg/100 kcal. Most preferably the nutritional composition comprises DHA in amount of about 25 mg/100 kcal and EPA in an amount of about 5.4 mg/100 kcal. In these embodiments the presence of ARA is optional. If present, the amount of ARA is preferably 12.5 to 50 mg, more preferably 25 to 33.5 mg and most preferably about 25 mg per 100 kcal. Preferably the weight ratio of DHA/ARA is from 0.9 to 2.

Preferably the weight ratio of DHA/EPA/ARA is 1: (0.19 -0.7) : (0.9-2.0). Such amounts and/or ratios of DHA, EPA and ARA are optimal for further improving the intestinal barrier function, for further improving the intestinal microbiota and/or for treatment or prevention of infections, in particular intestinal infections. The LC-PUFA may be provided as free fatty acids, in triglyceride form, in diglyceride form, in monoglyceride form, in phospholipid form, or as a mixture of one of more of the above. Suitable sources of these LC-PUFA are e.g. fish oil and oil from *Mortierella alpina.*

Preferably the nutritional composition according to the present invention comprises lipid, wherein the lipid comprise LC-PUFA selected from the group consisting of DHA, EPA and ARA, and wherein the sum of DHA, ARA and EPA is at least 1 wt.% based on total fatty acids, and wherein the lipid comprises DHA in amount of 0.4 to 1.0 wt.% based on total fatty acids, EPA in an amount of 0.09 to 0.4 wt.% based on total fatty acids and ARA in an amount of 0.25 to 1 wt.% based on total fatty acids. In this embodiment it is further preferred that the lipid comprises DHA in an amount of 0.5 to 0.7 wt.% based on total fatty acids, EPA in an amount of 0.1 to 0.2 wt.% based on total fatty acids and ARA in an amount of 0.5 to 0.7 wt.% based on total fatty acids. More preferably the lipid comprises DHA in an amount of at least 0.5 wt.%, EPA in an amount of at least 0.1 wt.% and ARA in an amount of at least 0.5 wt.%, all based on total fatty acids.

The combination of 2'-FL, butyrate, optional 3'-GL and LC-PUFA, in particular EPA, DHA and/or ARA, will have a further improved effect on health, in particular on improving the intestinal barrier function, on improving the immune system, on improving the intestinal microbiota and/or on the treatment or prevention of infections, in particular intestinal infections.

### Nutritional composition

The nutritional composition according to the present invention is not human milk.

The nutritional composition according to the present invention is for use in infants or young children.

The present nutritional composition preferably comprises lipid, protein and carbohydrate and is preferably administered in liquid form. The present nutritional composition may also be in the form of a dry food, preferably in the form of a powder which is accompanied with instructions as to mix said dry food, preferably powder, with a suitable liquid, preferably water. The present nutritional composition may thus be in the form of a powder, suitable to reconstitute with water to provide a ready-to-drink nutritional composition, preferably a ready-to-drink infant formula, follow-on formula or young child formula, more preferably a ready-to-drink infant formula or follow-on formula. The nutritional composition according to the invention preferably comprises other fractions, such as vitamins, minerals, trace elements and other micronutrients in order to make it a complete nutritional composition. Preferably infant formulae and follow-on formulae comprise vitamins, minerals, trace elements and other micronutrients according to international directives.

The present nutritional composition preferably comprises lipid, protein and digestible carbohydrate wherein the lipid provides 25 to 65% of the total calories, the protein provides 6.5 to 16% of the total calories, and the digestible carbohydrate provides 20 to 80% of the total calories. Preferably, in the present nutritional composition the lipid provides 30 to 55% of the total calories, the protein provides 7 to 9% of the total calories, and the digestible carbohydrate provides 35 to 60% of the total calories. For calculation of the % of total calories for the protein, the total of energy provided by proteins, peptides and amino acids needs to be taken into account.

Preferably the lipid provides 3 to 7 g lipid per 100 kcal, preferably 3.5 to 6 g per 100 kcal, the protein provides 1.6 to 4 g per 100 kcal, preferably 1.7 to 2.3 g per 100 kcal and the digestible carbohydrate provides 5 to 20 g per 100 kcal, preferably 8 to 15 g per 100 kcal of the nutritional composition. Preferably the present nutritional composition comprises lipid providing 3.5 to 6 g per 100 kcal, protein providing 1.7 to 2.3 g per 100 kcal and digestible carbohydrate providing 8 to 15 g per 100 kcal of the nutritional composition.

Preferably the lipid provides 2.5 to 6.5 g lipid per 100 ml, preferably 2.5 to 4 g per 100 ml, the protein provides 1 to 3 g per 100 ml, preferably 1 to 1.5 g per 100 ml and the digestible carbohydrate provides 3 to 13 g per 100 ml, preferably 5 to 10 g per 100 ml of the nutritional composition. Preferably the present nutritional composition comprises lipid providing 2.0 to 6.5 g per 100 ml, protein providing 1 to 3 g per 100 ml and digestible carbohydrate providing 5 to 10 g per 100 ml of the nutritional composition. Preferably the lipid provides 15 to 45 wt.%, preferably 20 to 30 wt.%, based on dry weight of the composition, the protein provides 8 to 20 wt.%, preferably 8.5 to 11.5 wt.%, based on dry weight of the composition and the digestible carbohydrates comprise 25 to 90 wt.%, preferably 40 to 75 wt.%, based on dry weight of the composition. Preferably the present nutritional composition comprises lipid providing 20 to 30 wt.%, protein providing 8.5 to 11.5 wt.% and digestible carbohydrate providing 40 to 75 wt.%, all based on dry weight of the composition.

The present composition preferably comprises lipids. Preferably the present composition comprises at least one lipid selected from the group consisting of vegetable lipids. Preferably the present composition comprises a combination of vegetable lipids and at least one oil selected from the group consisting of fish oil, algae oil, fungal oil, and bacterial oil. The lipid of the present nutritional composition preferably provides 3 to 7 g per 100 kcal of the nutritional composition, preferably the lipid provides 3.5 to 6 g per 100 kcal. When in liquid form, e.g. as a ready-to-feed liquid, the nutritional composition preferably comprises 2.0 to 6.5 g lipid per 100 ml, more preferably 2.5 to 4.0 g per 100 ml. Based on dry weight the present nutritional composition preferably comprises 15 to 45 wt.% lipid, more preferably 20 to 30 wt. Preferably the present nutritional composition comprises at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil.

The present nutritional composition preferably comprises protein. The protein used in the nutritional composition is preferably selected from the group consisting of non-human animal proteins, preferably milk proteins, vegetable proteins, such as preferably soy protein and/or rice protein, and mixtures thereof. The present nutritional composition preferably contains casein, and/or whey protein, more preferably bovine whey proteins and/or bovine casein. Thus in one embodiment the protein in the present nutritional composition comprises protein selected from the group consisting of whey protein and casein, preferably whey protein and casein, preferably the whey protein and/or casein is from cow's milk. Preferably the protein comprises less than 5 wt.% based on total protein of free amino acids, dipeptides, tripeptides or hydrolysed protein. The present nutritional composition preferably comprises casein and whey proteins in a weight ratio casein : whey protein of 10 : 90 to 90 : 10, more preferably 20 : 80 to 80 : 20, even more preferably 35 : 65 to 55 : 45.

In one embodiment, the protein used in the nutritional composition comprises hydrolysed protein, preferably the protein used in the nutritional composition is hydrolysed protein or in other words consists of hydrolysed protein. Hydrolysed protein may also comprise free amino acids. Preferably the hydrolysed protein comprises hydrolysed whey protein. In one embodiment, the protein used in the nutritional composition is free amino acids or in other words consists of free amino acids. Thus in a preferred embodiment, the nutritional composition according to the invention comprising 2'-FL and dietary butyrate and optionally also 3'GL, further comprises hydrolysed protein and/or free amino acids. Such compositions are preferably used for prevention or treating of allergy, more preferably for prevention or treating of cow's milk protein allergy.

The wt.% protein based on dry weight of the present nutritional composition is calculated according to the Kjeldahl-method by measuring total nitrogen and using a conversion factor of 6.38 in case of casein, or a conversion factor of 6.25 for other proteins than casein. The term 'protein' or 'protein component' as used in the present invention refers to the sum of proteins, peptides and free amino acids.

The present nutritional composition preferably comprises protein providing 1.6 to 4.0 g protein per 100 kcal of the nutritional composition, preferably providing 11.7 to 2.3 g per 100 kcal of the nutritional composition. A too low protein content based on total calories will result in less adequate growth and development in infants and young children. A too high amount will put a metabolic burden, e.g. on the kidneys of infants and young children. When in liquid form, as a ready-to-feed liquid, the nutritional composition preferably comprises 1.0 to 3.0 g, more preferably 1.0 to 1.5 g protein per 100 ml. Based on dry weight the present nutritional composition preferably comprises 8 to 20 wt.% protein, more preferably 8.5 to 11.5 wt.%, based on dry weight of the total nutritional composition.

The present nutritional composition preferably comprises digestible carbohydrate providing 5 to 20 g per 100 kcal, preferably 8 to 15 g per 100 kcal. Preferably the amount of digestible carbohydrate in the present nutritional composition is 25 to 90 wt.%, more preferably 8.5 to 11.5 wt.%, based on total dry weight of the composition. Preferred digestible carbohydrates are lactose, glucose, sucrose, fructose, galactose, maltose, starch and maltodextrin. Lactose is the main digestible carbohydrate present in human milk. The present nutritional composition preferably comprises lactose. Preferably the present nutritional composition does not comprise high amounts of carbohydrates other than lactose. Compared to digestible carbohydrates such as maltodextrin, sucrose, glucose, maltose and other digestible carbohydrates with a high glycemic index, lactose has a lower glycemic index and is therefore preferred. The present nutritional composition preferably comprises digestible carbohydrate, wherein at least 35 wt.%, more preferably at least 50 wt.%, more preferably at least 60 wt.%, more preferably at least 75 wt.%, even more preferably at least 90 wt.%, most preferably at least 95 wt.% of the digestible carbohydrate is lactose. Based on dry weight the present nutritional composition preferably comprises at least 25 wt.% lactose, preferably at least 40 wt.%, more preferably at least 50 wt.% lactose.

The present nutritional composition preferably comprises non-digestible oligosaccharides (NDO). The term "oligosaccharides" as used herein refers to saccharides with a degree of polymerization (DP) of 2 to 250, preferably a DP 2 to 100, more preferably 2 to 60, even more preferably 2 to 10. If oligosaccharide with a DP of 2 to 100 is included in the present nutritional composition, this results in compositions that may contain oligosaccharides with a DP of 2 to 5, a DP of 50 to 70 and/or a DP of 7 to 60. The term "non-digestible oligosaccharides" (NDO) as used in the present invention refers to oligosaccharides which are not digested in the intestine by the action of acids or digestive enzymes present in the human upper digestive tract, e.g. small intestine and stomach, but which are preferably fermented by the human intestinal microbiota. For example, sucrose, lactose, maltose and maltodextrins are considered digestible.

Preferably the present non-digestible oligosaccharides are soluble. The term "soluble" as used herein, when having reference to a polysaccharides, fibres or oligosaccharides, means that the substance is at least soluble according to the method described by L. Prosky et al., J. Assoc. Off. Anal. Chem. 71, 1017-1023 (1988).

The beta1,3'-galactosyllactose may be present in the nutritional composition according to the invention as such, or as part of a mixture of galacto-oligosaccharides (GOS), preferably beta-galacto-oligosaccharides (BGOS). In a preferred embodiment the beta1,3'-galactosyllactose is present as part of a mixture of galacto-oligosaccharides. In one embodiment, the amount of Gal (beta 1-3) - Gal (beta 1-4) - Glc is more than 20 wt.% based on total galacto-oligosaccharides.

Preferably the present nutritional composition also comprises fructo-oligosaccharides (FOS). The term "fructo-oligosaccharides" as used in the present invention refers to carbohydrates composed of over 50%, preferably over 65 % fructose units based on monomeric subunits, in which at least 50%, more preferably at least 75%, even more preferably at least 90%, of the fructose units are linked together via a beta-glycosidic linkage, preferably a beta-2,1 glycosidic linkage. A glucose unit may be present at the reducing end of the chain of fructose units. Preferably the fructo-oligosaccharides have a DP or average DP in the range of 2 to 250, more preferably 2 to 100, even more preferably 10 to 60. The term "fructo-oligosaccharides" comprises levan, hydrolysed levan, inulin, hydrolysed inulin, and synthesised fructo-oligosaccharides. Preferably the preparation comprises short chain fructo-oligosaccharides with an average degree of polymerization (DP) of 3 to 6, more preferably hydrolysed inulin or synthetic fructo-oligosaccharide. Preferably the preparation comprises long chain fructo-oligosaccharides with an average DP above 20. Preferably the preparation comprises both short chain and long chain fructo-oligosaccharides. Fructo-oligosaccharide suitable for use in the composition of the invention is also readily commercially available, e.g. RaftilineHP (Orafti). Preferably the nutritional composition according to the present invention comprises at least 25 mg FOS per 100 ml, more preferably at least 40 even more preferably at least 60 mg. Preferably the composition does not comprise more than 250 mg FOS per 100 ml, more preferably not more than 150 mg per 100 ml and most preferably not more than 100 mg per 100 ml. The amount of FOS is preferably 25 to 250 g fructo-oligosaccharides per 100 ml, preferably 40 to 150 g per 100 ml, more preferably 60 to 100 g per 100 ml. Preferably the nutritional composition according to the present invention comprises at least 0.15 wt.% FOS based on dry weight, more preferably at least 0.25 wt.%, even more preferably at least 0.4 wt.%. Preferably the composition does not comprise more than 1.5 wt.% FOS based on dry weight of the total composition, more preferably not more than 2 wt.%. The presence of FOS shows a further improved effect on the microbiota and its SCFA production.

Preferably the present nutritional composition comprises a mixture of galacto-oligosaccharides (including the beta1 ,3'-galactosyllactose) and fructo-oligosaccharides. Preferably the mixture of galacto-oligosaccharides and fructo-oligosaccharides is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when the galacto-oligosaccharides have a low average DP and fructo-oligosaccharides has a relatively high DP. Most preferred is a mixture of galacto-oligosaccharides with an average DP below 10, preferably below 6, and fructo-oligosaccharides with an average DP above 7, preferably above 11, even more preferably above 20.

In a preferred embodiment the present nutritional composition comprises a mixture of short chain (sc) fructo-oligosaccharides and long chain (Ic) fructo-oligosaccharides. Preferably the mixture of short chain fructo-oligosaccharides and long chain fructo-oligosaccharides is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharides with an average DP below 10, preferably below 6 and a fructo-oligosaccharides with an average DP above 7, preferably above 11, even more preferably above 20.

In another preferred embodiment the present nutritional composition comprises a mixture of short chain (sc) fructo-oligosaccharides and short chain (sc) galacto-oligosaccharides. Preferably the mixture of short chain fructo-oligosaccharides and short chain galacto-oligosaccharides is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharides and short chain galacto-oligosaccharides with an average DP below 10, preferably below 6.

The present nutritional composition preferably comprises 1.75 to 17.5 wt.% total non-digestible oligosaccharides, more preferably 2.8 to 10.5 wt.%, most preferably 4.2 to 7 wt.%, based on dry weight of the nutritional composition. Based on 100 ml the present nutritional composition preferably comprises 0.25 to 2.5 g total non-digestible oligosaccharides, more preferably 0.4 to 1.5 g, most preferably 0.6 to 1 g, based on 100 ml of the nutritional composition. A lower amount of non-digestible oligosaccharides will be less effective in improving the intestinal barrier function, whereas a too high amount will result in side-effects of bloating and abdominal discomfort. The total amount of non-digestible oligosaccharides includes galacto-oligosaccharides, including beta3'-GL, fructo-oligosaccharides and any additional non-digestible oligosaccharides that may further be present in the composition.

It is also important that the nutritional composition according to the present invention does not have an excessive caloric density, however still provides sufficient calories to feed the subject. Hence, the liquid food preferably has a caloric density between 0.1 and 2.5 kcal/ml, more preferably a caloric density of between 0.5 and 1.5 kcal/ml, even more preferably between 0.6 and 0.8 kcal/ml, and most preferably between 0.65 and 0.7 kcal/ml.

### Application

The present nutritional composition is preferably an infant formula, a follow-on formula or a young child formula. Examples of a young child formula are toddler milk, toddler formula and growing up milk. More preferably the nutritional composition is an infant formula or a follow-on formula. The present nutritional composition can be advantageously applied as a complete nutrition for infants. An infant formula is defined as a formula for use in infants and can for example be a starter formula, intended for infants of 0 to 6 or 0 to 4 months of age. A follow-on formula is intended for infants of 4 or 6 months to 12 months of age. At this age infants start weaning on other food. A young child formula, or toddler or growing up milk or formula is intended for children of 12 to 36 months of age. Preferably the present nutritional composition is an infant formula.

The infant formula, follow-on formula or young child formula may be in the form of a liquid, preferably a ready-to-drink liquid, or in the form of a powder. In one embodiment the infant formula, follow-on formula or young child formula is in the form of a powder, suitable to reconstitute with water to provide a ready-to-drink infant formula, follow-on formula or young child formula. It is to be understood that when the infant formula, follow-on formula or young child formula according to the invention is in the form of a powder, the amounts of all ingredients including non-digestible oligosaccharides, 2'-FL and 3'-GL in said formula are defined as the amounts that would be present after reconstitution of the powder with water, i.e. the amounts are defined in mg per 100 ml ready-to-drink formula.

The nutritional composition according to the invention is for use in providing nutrition to an infant or young child, preferably an infant, preferably up to 12 months of age.

The infant formula, follow-on formula or young child formula according to the invention is for use in providing nutrition to an infant or young child, preferably an infant, preferably up to 12 months of age. The preferred embodiments described above for the infant formula, follow-on formula and young child formula according to the invention also apply to the present infant formula for use, follow-on formula for use and young child formula for use.

The invention further relates to a composition comprising 2-'FL, dietary butyrate and optionally 3-'GL or the composition according to the invention, for use in the treatment or prevention of infections, in particular intestinal infections and/or for use in inducing oral tolerance to allergens.

As the nutritional composition of the invention has an improved effect on the intestinal barrier function, it will reduce the translocation of allergens, toxins and/or pathogens, and thereby will prevent or treat infections. As an improved effect on the intestinal alkaline phosphatase activity was also found, the nutritional composition will reduce the intestinal pathogens, thereby preventing and/or treating infections, in particular intestinal infections. Improvement of lactase maturation and intestinal cell proliferation is further indicative for an improved gut barrier maturation. Improvement in the microbiota, an increase in bifidobacteria, an enhanced acidification by fermentation, and reduction in pathogens was observed. Improvement of intestinal microbiota and/or immune system will furthermore beneficially prevent and/or treat infections, in particular intestinal infections. Effects on the immune system will have an effect on inducing oral tolerance to allergens.

Effects both in IL-10 as well as with CCL20 levels indicated an unexpected improved modulation in responsiveness of the human PBMC in the presence of a combination of 2'-FL and butyrate, which is even further improved when 3'-GL is present.

As the nutritional composition of the invention has an improved effect on decreasing the Th2 response, it thereby will prevent and/or treat allergy.

The nutritional composition according to the invention is preferably for use in providing nutrition to an infant or young child, preferably an infant, that suffers from allergy or has an increased risk of suffering from allergy.

Also disclosed, but not part of the present invention, is the use of the nutritional composition according to the present invention for providing nutrition to infants or young children, preferably for providing nutrition to infants.

### BRIEF DESCRIPTION OF THE FIGURES

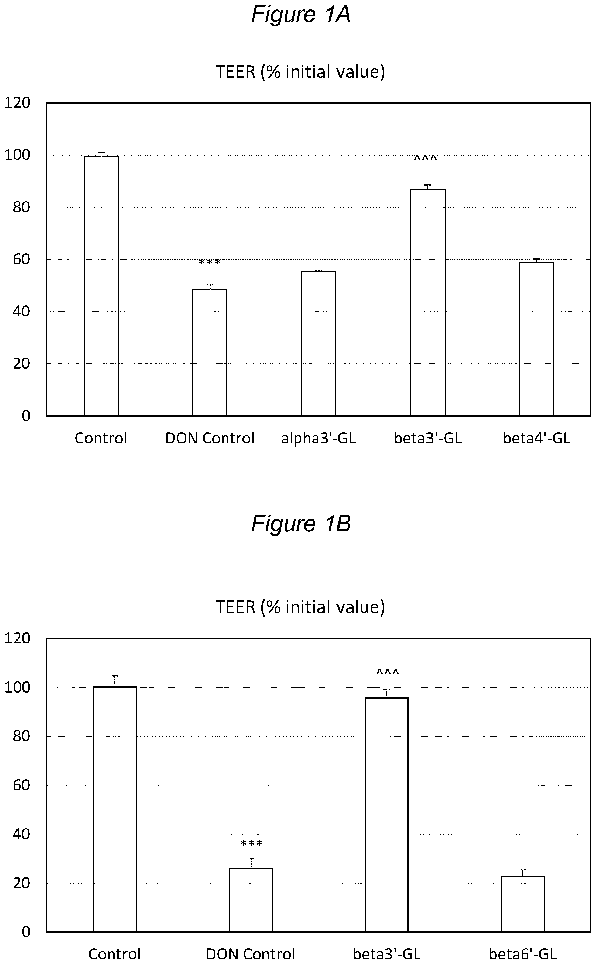

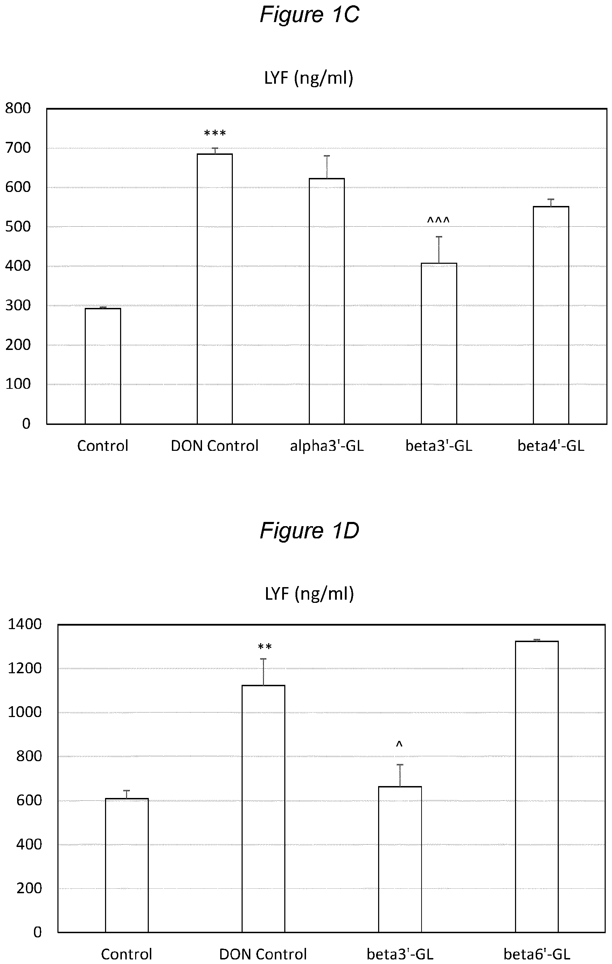

*Figure 1**:*
   Effects of different galactosyllactoses (GLs) on the DON-induced impairment of the Caco-2 cell monolayer integrity. Figures 1A and 1B show the transepithelial electrical resistance (TEER) for different GLs. Figures 1C and 1D show the translocation of lucifer yellow (LYF) to the basolateral compartment. TEER was expressed as a percentage of the initial value and LYF was expressed in ng/cm² × h, i.e. in ng/ml. alpha3'-GL is Gal (alpha 1-3) - Gal (beta 1-4) - Glc; beta3'-GL is Gal (beta 1-3) - Gal (beta 1-4) - Glc; beta4'-GL is Gal (beta 1-4) - Gal (beta 1-4) - Glc'; beta6'-GL is Gal (beta 1-6) - Gal (beta 1-4) - Glc. Data are the mean ± s.e. *: p < 0.05 compared to control, **: p < 0.01 compared to control, ***: p < 0.001 compared to control, ^: p < 0.05 compared to DON control, ^^ p< 0.01 compared to DON Control, ^^^ p< 0.001 compared to DON Control.
*Figure 2**:*
   Different effects of GLs on the DON-induced increase in IL8 release by Caco-2 cells. IL-8 secretion is expressed as pg/ml as mean ±s.e. alpha3'-GL is Gal (alpha 1-3) - Gal (beta 1-4) - Glc; beta3'-GL is Gal (beta 1-3) - Gal (beta 1-4) - Glc , beta4'-GL is Gal (beta 1-4) - Gal (beta 1-4) - Glc , beta6'-GL is Gal (beta 1-6) - Gal (beta 1-4) - Glc. Data are the mean ± s.e. *: p < 0.05 compared to control, **: p < 0.01 compared to control, ***: p < 0.001 compared to control, ^: p < 0.05 compared to DON control, ^^ p< 0.01 compared to DON Control, ^^^ p< 0.001 compared to DON Control.

### EXAMPLES

### Example 1: Infant formula with 2'-FL and dietary butyrate improve intestinal alkaline phosphatase expression

Two infant formulae were subjected to an *in vitro* digestion step and after the *in vitro* digestion step the effect on intestinal barrier maturation was examined, in particular the maturation of alkaline phosphate (AP). AP is an intestinal enzyme that is expressed and secreted by enterocytes and used as differentiation marker. AP plays a pivotal role in intestinal homeostasis and innate immune defence by dephosphorylating harmful substances such as microbial ligand lipopolysaccharide (endotoxin).

The control infant formula was a non-fermented infant formula supplemented with non-digestible oligosaccharides (scGos/IcFOS) in an amount of 0.8 mg/100 ml when in ready to drink form. The scGOS being derived from Vivinal GOS and the IcFOS being derived from RaftilineHP. The fat component being mainly vegetable oils, fish oil and microbial oil (source of arachidonic acid). The amount of butyric acid was below 0.05 wt.% based on total fat.

The active infant formula was the partly fermented infant formula similar to example 8, i.e. additionally containing 0.1 g 2'-FL, the lipid component comprising about 50 wt.% of bovine milk fat, and having about 1.5 wt.% of butyric acid based on total fatty acids, about 3.4 g fat per 100 ml about 0.28 wt.% lactic acid based on dry weight, and about 25 mg 3'-GL per 100 ml when in ready to drink form.

### In vitro digestion:

Infant formulae were prepared at 13.7% (w/v) in MiliQ water and 35 ml was transferred to bio-reactors in a computer controlled semi-dynamic gastrointestinal model simulating infant conditions. Each reactor was equipped with a pH electrode and four dosing lines. Each dosing line was connected to a pump adding either; a) hydrochloric acid 0.25M and b) Sodium bicarbonate 0.5 M for pH control or c) Simulated Gastric Fluid (SGF), d) Simulated Intestinal Fluid (SIF). The pH was controlled by standardizing to 6.8 at the start of digestion, then lowering the pH gradually during a 2-hour gastric phase to 4.3. In the intestinal phase of digestion, the pH is gradually raised from 6.5 to 7.2 over 2 hours. At t=0 (the start of digestion), 5.8 ml of Simulated Salivary Fluid (100mM NaCl, 30mM KCI, 1.4 mM CaCl₂, 14mM NaHCO₃, 0.6 mg/ml α-amylase from *Aspergillus oryzae* (SIGMA, A9857)) was added as a bolus. From t=0 onwards 12.25 ml of SGF (100 mM NaCl, 30mM KCI, 1.4 mM CaCl₂, 50 mM Sodium acetate, 0.125 mg/ml pepsin from porcine gastric mucosa (SIGMA, P7012), and 0.05 mg/ml Lipase from *Rhizopus oryzae,* Amano) was gradually added until t=120 (the end of the gastric phase). The consecutive intestinal phase started with the pH being increased to 6.5, and the gradual addition of 31.5 ml SIF (100 mM NaCl, 10 mM KCI, 1.7 mM CaCl₂, 0.17 mg/ml trypsin from bovine pancreas (SIGMA, T9201), 0.18 mg/ml chymotrypsin from bovine pancreas (SIGMA, C4129), 0.09 mg/ml pancreatic Lipase from porcine pancreas (SIGMA, L0382), 1.42 mg/ml Taurocholate (SIGMA, 86339) and 0.6 mg/ml Tauroursodeoxycholate (SIGMA, T0266)). At the end of simulated gastro-intestinal digestion a 5 ml sample was taken, mixed with 5 ml enzyme inhibitor buffer (0.1 M sodium phosphate, pH 5.5, 0.58 mg/ml trypsin-chymotrypsin inhibitor from *Glycine max* (SIGMA, T9777), 34.5 µg/ml Orlistat (SIGMA, O4139)) snap frozen and stored at -20°C until further use.

### Cell differentiation

Cells from the enterocyte-like and brush border expressing human intestinal cell line C2BBe1 (ATCC^{®} CRL-2102 ^{™}) were seeded at 5000 cells/well in 96-wells Nunc^{™} Edge plates and grown to confluency in Dulbecco's Modified Eagle's Medium, (Catalog No. 30-2002) with 10% fetal calf serum, 1% penicillin/streptomycin and 0.01 mg/ml human transferrin. After reaching confluency, culture medium was replaced with predigested infant formula diluted in culture medium without fetal calf serum at final concentrations of 0.34 %, 0.17 % and 0.08 5% (w/v) in quadruplicates and incubated at 37 °C, 5 % CO₂ for 96 hours, refreshing with the diluted predigested infant formula after 48 hours. At the end of the incubation period, 50 µl of culture medium was collected per well, the quadruplicates were pooled and stored at -20 °C until measurement of the AP activity. Then, all wells were washed with ice-cold Phosphate Buffered Saline and to each well, 100 µl of 50 mM Tris-HCL, 150 mM NaCl, 0,5% triton-100 at pH 7.0 was added. After 30 min incubation on ice, cell lysates were collected and protein content was determined using Thermo Fischer, Pierce BCA Protein Assay Kit according to the manufacturer's instructions. APactivity was determined by Biovision Alkaline Phosphatase Activity Colorimetric Assay Kit, according to the manufacturer's instructions. AP activity was expressed as Units/mg protein

### Results

The AP activity was statistically significantly increased (p<0.05, t-test) in the enterocytes that were treated with the predigested infant formula of the invention, when compared to the enterocytes treated with predigested control formula. This effect was dose dependent and significantly different at all concentrations tested. The increase in extracellular AP activity compared to the control formula was 43%, 36% and 32% at infant formula concentrations of 0.34, 0.17 and 0.085% (w/v), respectively, see Table 1. This increase in extracellular AP activity is indicative for an improved intestinal barrier function maturation and an improved defense against intestinal pathogenic bacteria.

**Table 1: AP activity of intestinal enterocytes exposed to predigested control or experimental formula in mU/mg protein.**

| Dilution (x) | IF concentration (g/100 ml) | Control formula | | Test formula | | |
|---|---|---|---|---|---|---|
| | | Mean | SEM | Mean | SEM | P* |
| 40 | 0.34 | 0.84485 | 0.08992 | 1.20802 | 4.601E-02 | 0.023 |
| 80 | 0.17 | 1.16073 | 0.05346 | 1.57569 | 6.284E-02 | 0.007 |
| 160 | 0.085 | 1.49291 | 0.11494 | 1.96274 | 5736E-02 | 0.022 |

| | | | | | | |
|---|---|---|---|---|---|---|
| * p value determined by t-test, 2-tailed, two-sample equal variance. | | | | | | |

### Example 2 Infant formula with 2'-FL and dietary butyrate improve intestinal lactase expression and cell proliferation

The nutritional compositions of example 1 were tested in a similar experiment as example 1. Instead of 13.7, 13.6% (w/v) of the formula was used. Instead of lipase from Rhizopus oryzae, rabbit lipase was used at 16.6 mg/ml (Germ, REG.340) in the gastric phase. During the intestinal phase, 0.06 mg/ml pancreatic Lipase from porcine pancreas (SIGMA, L0382), and 3.5 mg/ml porcine pancreatic lipase (SIGMA L0126) was used instead of 0.09 mg/ml pancreatic Lipase from porcine pancreas (SIGMA, L0382).

Lactase activity was measured by mixing 30 µl of cell lysate with 30 µl assay buffer (maleic acid 0.625 M, lactose 0.12 M, pH 6.0) and incubated at 37°C for 4 hours, the resulting glucose was quantified. Lactase activity was expressed as µmol glucose/min/mg.

It was found that the lactase activity was significantly increased when cells were treated with the predigested experimental test infant formula compared to predigested control formula, see Table 2.

**Table 2: Lactase activity of intestinal enterocytes exposed to predigested control or experimental formula in mU/mg protein.**

| Dilution (x) | IF concentration (g/100 ml) | Control formula | | Test formula | | |
|---|---|---|---|---|---|---|
| | | Mean | SEM | Mean | SEM | P* |
| 80 | 0.17 | 0.63 | 0.02 | 0.816 | 0.008 | 0.001 |
| 160 | 0.085 | 0.69 | 0.01 | 0.831 | 0.037 | 0.020 |

| | | | | | | |
|---|---|---|---|---|---|---|
| * p value determined by t-test, 2-tailed, two-sample equal variance. | | | | | | |

Lactase activity increases in differentiating enterocytes, followed by an increase in sucrase activity after which brush border lactase activity starts dropping off. Since the cells did not show sucrase activity at the time of measurement (data not shown), an increased lactase activity is thus indicative for a more differentiated cell state.

### Cell Proliferation test

Crypt-like human colon carcinoma HT-29 cells were seeded at 5.10⁴ in 96-wells Nunc^{™} Edge plates in DMEM with 10% FCS, 1% penicillin/streptomycin and 1g/L galactose. Cells were allowed to adhere for 30 hours after which medium was replaced with digested IF diluted in culture medium without fetal calf serum at final concentrations of 0.23%, 0.17% and 0.085% (w/v) in triplicates. Different cell proliferation rates resulted in different cellular protein contents after 72 hours incubation, these were measured by lysing cell followed by protein content determination with Thermo Fischer, Pierce BCA Protein Assay Kit according to the manufacturer's instructions.

Cell proliferation was significantly increased as shown by an increased cellular protein content of cells treated with the predigested experimental, test infant formula compared to predigested control formula (Table 3).

**Table 3: Proliferation (cellular protein ug/well) of intestinal enterocytes exposed to predigested control or experimental formula in mU/mg protein.**

| Dilution (x) | IF concentration (g/100 ml) | Control formula | | Test formula | | |
|---|---|---|---|---|---|---|
| | | Mean | SEM | Mean | SEM | P* |
| 80 | 0.17 | 21.2 | 0.1 | 23.9 | 0.6 | 0.011 |
| 160 | 0.085 | 21.4 | 0.3 | 23.9 | 0.6 | 0.018 |

| | | | | | | |
|---|---|---|---|---|---|---|
| * p value determined by t-test, 2-tailed, two-sample equal variance. | | | | | | |

To achieve its function as a barrier to the external environment, the gut epithelium must be continuously renewed. The growth and renewal of gut epithelial cells depends on proliferating cells in the intestinal crypts. Stimulation of the cell proliferation rate thus is expected to support the gut barrier function.

### Example 3: 3'-GL and 2'-FL protect against intestinal barrier disruption and prevents permeability increase.

Beta1 ,3'-galactosyl-lactose (beta3'-GL), beta1 ,4'-galactosyllactose (beta4'-GL) and beta1 ,6'-galactosyl-lactose (beta6'-GL) were obtained from Carbosynth (Berkshire, UK). Alpha1,3'-galactosyl-lactose (alpha3'-GL) was obtained from Elicityl (Crolles, France). Purified deoxydivalenol (DON) (D0156; Sigma Aldrich, St Luis, MO, USA) was dissolved in pure ethanol and stored at -20 °C. Human epithelial colorectal adenocarcinoma (Caco-2) cells were obtained from American Type Tissue Collection (Code HTB-37) (Manasse, VA, USA, passage 90-102).

Caco-2 cells were used according to established methods. In brief: cells were cultured in Dulbecco's modified Eagle medium (DMEM) and seeded at a density of 0.3×10⁵ cells into 0.3 cm² high pore density (0.4 µm) inserts with a polyethylene terephthalate membrane (BD Biosciences, Franklin Lakes, NJ, USA) placed in a 24-well plate. The Caco-2 cells were maintained in a humidified atmosphere of 95 % air and 5 % CO₂ at 37 °C. After 17-19 days of culturing, a confluent monolayer was obtained with a mean transepithelial electrical resistance (TEER) exceeding 400 Ω cm² measured by a Millicell-Electrical Resistance System voltohm-meter (Millipore, Temecula, CA, USA).

Caco-2 cell monolayers were thus grown in a transwell system, which is a model for intestinal barrier function. The monolayers were pretreated for 24 h with different GLs, including beta3'-GL, alpha3'-GL, beta4'-GL and beta6'-GL in a concentration of 0.75 wt.% of the GL, before being exposed to the fungal toxin deoxynivalenol (DON), which is a trigger and model compound to impair intestinal barrier. DON was diluted to a final concentration of 4.2 µM in complete cell medium and added to the apical side as well as to the basolateral side of the transwell inserts. This DON concentration did not affect the viability of the Caco-2 cells. Incubation with DON was 24 h.

Measurements of the transepithelial electrical resistance (TEER) and lucifer yellow (LY) permeability were conducted to investigate barrier integrity. For TEER measurements a Millicel-ERS voltohmmeter connected to a pair of chopsticks electrodes was used to measure the TEER values. Results are expressed as a percentage of the initial value. For paracellular tracer flux assay the membrane impermeable lucifer yellow (LY) (Sigma, St Luis, MO, USA) was added in a concentration of 16 µg/ml to the apical compartment in the transwell plate for 4 h, and the paracellular flux was determined by measuring the fluorescence intensity in the basolateral compartment with a spectrophotofluorimeter (FLUOstar Optima, BMG Labtech, Offenburg, Germany) set at excitation and emission wavelengths of 410 and 520 nm, respectively. Release of interleukin-8 (IL-8 or CXCL8), which is a typical marker for inflammation, was quantified in the medium of the apical side and the basolateral side of the Caco-2 transwell inserts in response to the treatments. CXCL8 concentrations were measured by using the human IL-8 ELISA assay (BD Biosciences, Pharmingem, San Diego, CA, USA) according to manufacturer's instructions. For more details on materials and methods see Akbari et al, 2016, Eur J Nutr. 56(5):1919-1930.

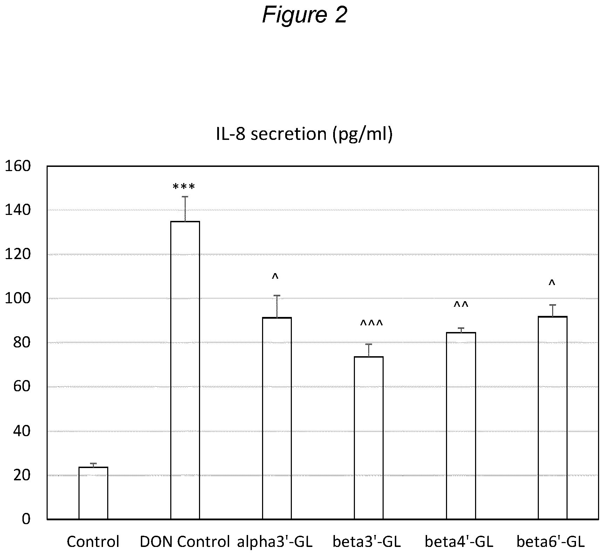

The results are shown in Figure 1 A, B, C and D and in Figure 2. Figure 1 shows the effects of different galactosyllactoses (GLs) on the DON-induced impairment of the Caco-2 cell monolayer integrity.

Figures 1A and 1B show the transepithelial electrical resistance (TEER) for different GLs. Figures 1C and 1D show the translocation of lucifer yellow (LYF) to the basolateral compartment. TEER was expressed as a percentage of the initial value and LYF was expressed in ng/cm²×h. alpha3'-GL is Gal (alpha 1-3) - Gal (beta 1-4) - Glc; beta3'-GL is Gal (beta 1-3) - Gal (beta 1-4) - Glc; beta4'-GL is Gal (beta 1-4) - Gal (beta 1-4) - Glc; beta6'-GL is Gal (beta 1-6) - Gal (beta 1-4) - Glc. Data are the mean ± s.e. *: p < 0.05 compared to control, **: p < 0.01 compared to control, ***: p < 0.001 compared to control, ^: p < 0.05 compared to DON control , ^^ p< 0.01 compared to DON Control, ^^^ p< 0.001 compared to DON Control.

As can be seen from Figures 1A-D, the presence of DON disrupted the barrier function as shown by a decreased TEER value and an increased LY flux for the DON-control samples. Additionally, the presence of DON increased CXCL8 (IL-8) release, as shown in Figure 2. Figures 1A-D further show that the presence of beta3'-GL prevented the DON-induced loss of epithelial barrier integrity as measured by increased TEER values and a reduction in the DON-affected LY flux across the intestinal epithelial monolayer.beta4'-GL and beta6'-GL did not show a significant effect on the intestinal epithelial barrier function. Interestingly, beta3'-GL, i.e. the galactosyl-lactose with a β1-3 glycosidic linkage, was effective in protecting the intestinal barrier function, whereas alpha3'-GL, i.e. the galactosyl-lactose with an α1-3 glycosidic linkage, did not prevent the DON-induced disrupted intestinal barrier. In contrast, all galactosyl-lactoses were able to decrease the DON-induced IL-8 release, as is shown in Figure 2. These results are indicative for the specific effect of beta3'-GL (herein also referred to as beta1,3'-galactosyllactose or Gal (beta 1-3) - Gal (beta 1-4) - Glc) on protecting the intestinal epithelial barrier function, in particular under conditions of challenges, which goes beyond and/or is independent of an effect on preventing an inflammatory response, and/or of an effect on or via the microbiota. These results are thus indicative of an effect that beta3'-GL has on increasing the intestinal barrier function and/or on the prevention and/or treatment of intestinal barrier disruption. In addition, these results are indicative of an effect of beta3'-GL on the treatment, prevention and/or alleviation of a toxin exposure associated condition in a subject, in particular when the toxin is a tricothecene toxin, and more in particular when the toxin is deoxynivalenol.

In a separate experiment the effect of 2'fucosyllactose on TEER and LYF flux was determined in the same model. 2'-FL was tested in a concentration of 1 mg/ml, and was found to statistically significantly prevent the DON induced reduction in TEER and increase in LYF, see Table 4. This is indicative for the advantageous effect 2'-FL has on the intestinal barrier function. Therefore this example is indicative of a further improved effect on the intestinal barrier function in a composition, when combining 2'-FL and beta3'-GL.

**Table 4: Effect of 2'-FL on the DON-induced impairment of the Caco-2 cell monolayer integrity.**

| | TEER (% of initial value) | Lucifer yellow flux in ng/ml |
|---|---|---|
| | Mean (s.e.) | (cm²×h) |
| Control | 101.3 (0.379) | 302.7 (7.325) |
| Control with DON | 34.33 (1.088)^{∗∗∗} | 530.8 (3.975)^{∗∗∗} |
| DON with 2'-FL (1 mg/ml) | 42.79 (0.844)^^ | 446.4 (8.302)^ |

| | | |
|---|---|---|
| *** p < 0.001 compared to control without DON. ^^ p < 0.01 compared to control with DON, ^p < 0.05 compared to control with DON | | |

### Example 4: Butyrate improves intestinal barrier function

The effect of butyrate on the intestinal barrier function was examined

### Methods

T84 human intestinal epithelial cells are commonly used to study intestinal barrier integrity in vitro. T84 cells (ATCC, USA) were cultured on 12 mm transwell inserts (0.4 µm, Corning Costrar, USA) in DMEM-F12 glutamax with penicillin-streptomycin (100 IU/ml), supplemented with 5% FBS-HI. T84 cells were used 14 days after reaching confluence. Monolayers of T84 cultured on transwell filters were preincubated for 48 h with or without butyrate. These samples were subsequently incubated for an additional 48 h in the presence of IL-4 (25 ng/ml). IL-4 was added to the basolateral compartment; medium and additives were changed every 24 h.

Epithelial barrier integrity was assessed by measuring transepithelial resistance (TEER; Ω × cm²) with the epithelial volt-ohm meter (EVOM; World Precision Instruments, Germany).

Results are shown in Table 5 where relative TEER values are presented. The 48h and 96h column is the TEER increase relative to t=0 value. IL-4 treatment disrupted the intestinal barrier function; however in the presence of butyrate this disruption was ameliorated.

**Table 5: Effect of butyrate on the intestinal barrier function.**

| Butyric acid concentration (mM) | % TEER at 48 h | % TEER at 96 h (IL-4) |
|---|---|---|
| - | 17 (12) | 0(4) |
| 4 | 79 (25) | 26 (16) |

Therefore this example is indicative of a further improved effect on the intestinal barrier function in a composition, when combining 2'-FL and dietary butyrate and optionally beta3'-GL.

### Example 5: 2'-FL and (3'-GL and/or butyrate) effect the immune system differently

Immune cell activation and responses were determined by culturing human peripheral blood mononuclear cells (PBMCs) in the presence or absence of 2'-FL, 3'-GL and butyric acid with and without T cell specific stimulation.

### Material and Methods

Isolation of PBMC from healthy donors: Human peripheral blood mononuclear cells (PBMC) from healthy donors were isolated from buffy coats (Sanquin, Amsterdam, the Netherlands). PBMC were obtained by centrifugation using Leucosep tubes (Greiner Bio-One). PBMC were collected and washed in PBS (Gibco, Thermo Fisher Technologies) + 2% heat-inactivated FCS (Invitrogen), followed by hypotonic lysis of erythrocytes with sterile lysis buffer (0.15 M NH₄Cl, 0.01 M KHCO₃ and 0.1 mM EDTA, pH of 7.4 at 4 °C, all from Merck, Darmstadt, Germany). After lysis, the PBMC were resuspended in freezing medium (70% RPMI 1640 medium (Gibco, Thermo Fisher Technologies) supplemented with 10% heat-inactivated FCS and 100 U/ml penicillin-streptomycin, 20% heat-inactivated FCS and 10% DMSO (Sigma)) and cryopreserved.

PBMC activation model: PBMC (0.2·10⁶ cell/well) were cultured in 96-well flat bottom plates (Corning). For 24 hours the cells were pre-incubated with 2'-FL (Jennewein), 3'-GL (0-0.3% w/v; Carbosynth) or sodium butyrate (0.2 mM; Sigma Aldrich) and combinations thereof. Subsequently, the cells were CD3/CD28-activated (Pelicluster CD3 and Pelicluster CD28, Sanquin) for an additional 24 hours. After incubation, IFNγ was measured by ELISA in the supernatants (see below). To determine cell activity after stimulation, PBMC were incubated with cell proliferation reagent WST-1 (10 µl; Roche) and/or 10% Triton (5 µl; negative control). After 3 hours, absorbance was measured at OD450nm and OD650nm and cell activity was calculated according to manufacturer's instructions.

IFNγ production PBMC: PBMC were incubated with indicated reagents and after incubation the supernatants were collected and mediator levels were measured using human IFNγ ELISA kits (R&D Systems Europe Ltd.) according to manufacturer's instructions.

Cytokine production of PBMC: PBMC were incubated with indicated reagents. After incubation, the supernatants were collected and IL2, IL6, IL10, IL13, IL21, TNFα, IFNγ, MIF, CCL1, CCL13, CCL17, CCL20, CCL22 and CXCL8-11 levels were measured by conducting a validated multiplex immunoassay based on Luminex technology (xMAP, Luminex Austin TX USA). Acquisition was conducted with the Biorad FlexMAP3D (Biorad laboratories, Hercules USA) in combination with xPONENT software version 4.2 (Luminex). Data was analyzed by 5-parametric curve fitting using Bio-Plex Manager software, version 6.1.1 (Biorad).

After PBMC stimulation, cell culture supernatants were collected, after which cytokine responses were measured in order to test immune responsiveness of the cells. The levels of cytokines measured in stimulated conditions were corrected for the (low) levels of the cytokines measured in non-stimulated conditions. In addition, since each donor is reacting in its own efficiency onto the T cell stimulus, we calculated the individual index of cytokine response by dividing the intervention induced response by the basal stimulated response.

Generally IL2, IL6, TNF-alpha, CCL1, CCL17, and CCL20 are considered to be associated with inflammation and/or proliferation. IFN-gamma, CXCL9, CXCL10, and CXCL11 are considered to be associated with a Th1 response. IL13, CCL13, and CCL22 are considered to be associated with a Th2 response. IL10 and Galectin-9 are considered to be associated with a Treg effect and IL21 is associated with a B-cell effect.

Statistical analysis: Comparison between CD3/CD28 stimulated and controls were made using paired one-tailed (Wilcoxon) t test, p <0.05 was considered significantly different.

Relative mean ± SEM from the measured and calculated values in stimulated condition were statistically tested using paired two-tailed (Wilcoxon) t test p <0.05 was considered significantly different. The calculation values of the combined effect of the single ingredients was based on the per donor measured values.

Immune cell activity as measured by WST was significantly increased after 24h by the addition of 2'-FL, whereas a decrease in activation was detected upon addition of 3'-GL The addition of butyrate, did not influence immune cell metabolic activity neither in no-stimulated conditions, nor under T cell stimulated conditions (CD3/CD28).

The addition of 2'-FL altered the cytokine response, whereas the addition of 3'-GL did not result in the same changes. Interestingly the addition of 3'-GL with the 2'-FL seemed to boost the performance of 2'-FL significantly. Moreover, the difference that was found between the response derived from 3'-GL and 2'-FL and butate on the metabolic activity of the cells vs the IFN-gamma production, is indicative for other immune responses to be indicted.

Overall it is concluded that the total pool of isolated human PBMCs is a diverse pool of immune cells, which respond directly and differently to provided HMOs. Although cells become more metabolically active, the cytokine production in the presence of 2'-FL is not equal to the cytokine production in the presence of 3'-GL, suggesting differential immune reactive responses. The same was observed for the interaction with 2'-FL and butyrate.

### Results on 2'-FL and butyric acid

Interleukin-10 (IL10) is not a cell type-specific cytokine but is broadly expressed by many immune cells. The induction of IL10 often occurs together with other pro-inflammatory cytokines, although the pathways that induce IL10 may negatively regulate these pro-inflammatory cytokines. IL10 has a central role in infection by limiting/regulating the immune response to pathogens and thereby preventing damage to the host. Therefore, IL10 is generally regarded as a regulatory cytokine. IL 10 levels were measured in peripheral blood mononuclear cell (PBMC) cultures from 10 human donors.

**Table 6: IL10 levels in PBMC under unstimulated condition, depicted as relative (normalized) values compared to blanc, thereby correcting for donor variation**

| | Relative IL10 level, mean (se) |
|---|---|
| blanc | 1 (0) |
| 0.2% w/v 2'-FL | 3.361 (1.867) |
| 0.2 mM butyrate | 1.911 (0.203) |
| 0.2% w/v 2'-FL + 0.2 mM butyrate | 14.77 (5.074)* |
| Observed value | |
| 0.2% w/v 2'-FL + 0.2 mM butyrate | 4.272 (0.643) |
| Calculated value | |

| | |
|---|---|
| *: paired two-tailed (Wilcoxon) t test, p <0.05 when compared to the calculated value. | |

In human PBMCs co-cultured with 0.2% 2'-FL a significantly (p<0.05) increased level of IL10 was detected as compared to blanc control, whereas the addition of butyrate did not have an effect on IL10 levels. The combination 0.2% 2'-FL and 0.2 mM butyrate significantly increased IL10 levels as compared to the blanc control and to 0.2 mM butyrate. Interestingly the combination of 2'-FL and butyrate increased the IL10 to higher levels than theoretically can be expected based on individual components, see Table 6. This is indicative for an unexpected, beneficial increased regulatory capacity of the human PBMC in the presence of a combination of 2'-FL and butyrate when compared to the single ingredients.

In general, CCL20 and CCR6 play a role in the recruitment of immature DCs and their precursors to sites of potential antigen-entry. Depending on the tissue microenvironment (e.g. local presence of TGF-beta, IL10 or IL15), immune cells may acquire functional CCR6 and hence migrate to sites of CCL20 production. CCL20 is shown to rapidly induce firm adhesion of subsets of freshly isolated T-lymphocytes to intercellular adhesion molecule-1. Regulation can therefore be obtained through modulation of CCL20 in un-stimulated conditions.

**Table 7. CCL-20 levels in unstimulated condition depicted as relative values, thereby correcting for donor variation**

| | Relative CCL 20 mean (s.e.) |
|---|---|
| blanc | 1 (0) |
| 0.2 % w/v 2-'FL | 2.586 (0.281) |
| 0.1 w/v% 3'-GL | 1.164 (0.212) |
| 0.2 mM butyrate | 1.053 (0.127) |
| 0.2 w/v% 2'-FL + 0.2 mM butyrate | 4.955 (1.206)** |
| Observed value | |
| 0.2 w/v% 2'-FL+ 0.1 w/v % 3'-GL+ 0.2 mM butyrate | 8.127 (2.264)* |
| Observed value | |
| 0.2 w/v % 2-FL + 0.2 mM butyrate | 2.639 (0.347) |
| Calculated value | |
| 0.2 w/v% 2'-FL + 0.1 w/v% 3'-GL+ 0.2 mM butyrate | 2.803 (0.524) |
| Calculated value | |

| | |
|---|---|
| paired two-tailed (Wilcoxon) t test: * p<0.05, ** p<0.01 when compared with the calculated value. | |

In human PBMCs exposed to 2'-FL an increased level of CCL20 was detected as compared to blanc control, whereas the addition of butyrate or 3'-GL alone did not have a statistically significant effect on CCL20 levels. Incubation of human PBMCs with the combination of 2'-FL and butyrate induced significantly higher levels of CCL20 compared to the blanc and butyrate alone. The further presence of 3'-GL in this combination of 2'-FL and butyrate further enhanced the CCL20 levels. Unexpectedly, the observed levels of the combination of 2'-FL and butyrate was significantly higher than can be calculated based on the single ingredients. This was also the case when the observed value of the combination of 2'-FL, butyrate and 3'-GL was compared with the theoretically calculated value based on the single ingredients. see Table 7.

These data indicate that the addition of 2'-FL, and butyrate influence immune responsiveness of human PBMCs. The further presence of 3'-GL further improves the immune response. The total pool of isolated human PBMCs is a diverse pool of immune cells, which respond directly and differently to provided HMOs. Changes as detected both in IL-10 as well as with CCL20 levels are suggestive for an unexpected improved modulation in responsiveness of the human PBMC in the presence of a combination of 2'FL and butyrate, which is even further improved when 3'GL is present.

### Results on 2-'FL, 3'-GL and their combination

The effect of coculturing with 2'FL and 3'GL and their combination on PBMC cultures from 10 human donors was studied. First the effect of T-cell specific stimulation via CD3/CD28 was determined.

After stimulation of the human PBMCs, cell culture supernatants were collected, after which cytokine responses were measured in order to test immune responsiveness of the cells. Upon T-cell specific stimulation with CD3/CD28 several cytokines were detected within the cell supernatants using Luminex technology. The Th2 type of cytokines IL-4 and IL-13, chemokines CCL17 were significantly increased showing a robust T cell stimulation (Table 8).

**Table 8. Cytokine IL-4, IL-13 and chemokine CCL17 levels (pg/ml) as measured in cell culture supernatants of PBMCs after stimulation with CD3/CD28 as compared to unstimulated conditions.**

| | Unstimulated | Stimulated CD3/CD28 |
|---|---|---|
| | Mean (s.e.) | Mean (s.e.) |
| IL-4 | 1.117 (0.062) | 25.83 (5.45)*** |
| IL-13 | 6 (0) | 50.25 (9.13)*** |
| CCL17 | 1.80 (0.267) | 83.97 (19.89)*** |

| | | |
|---|---|---|
| Paired one-tailed (Wilcoxon) t test *p<0.05, **p<0.01, ***p<0.001, ****p<0.0001 stimulated vs unstimulated | | |

Subsequently, in order to test the direct effect of specific compounds on PBMC activity, the cells were activated with CD3/CD28 for 24 hours after a pre-incubated for 24h with either 2'-FL, 3'-GL and combinations thereof. In addition, since each donor is reacting in its own efficiency onto the T cell stimulus, the individual index of cytokine response was calculated by dividing the intervention induced response by the basal stimulated response (the blanc is set at 1). In this way the intervention within 10 different donors has been studied.

IL-4 and IL-13 are closely related cytokines, known to regulate many aspects of allergic inflammation. They play important roles in regulating the responses of lymphocytes, myeloid cells, and non-hematopoietic cells. For example; in T-cells, IL-4 induces the differentiation of naive CD4 T cells into Th2 type of T cells, in B cells, IL-4 drives the immunoglobulin (Ig) class switch to IgG1 and IgE, and in macrophages, IL-4 and IL-13 induce alternative macrophage activation.

**Table 9: Relative level of IL-4, IL-13 and CCL17 in CD3/CD28 stimulated condition with 2'-FL and 3'-GL, or the combination.**

| | IL-4 | IL-13 | CCL17 |
|---|---|---|---|
| | Mean (s.e.) | Means (s.e.) | Mean (s.e) |
| blanc | 1 (0) | 1 (0) | 1 (0) |
| 0.2 wt% 2'-FL | 0.9887 (0.0517) | 1.039 (0.080) | 1.061 (0.117) |
| 0.1 wt% 3'-GL | 0.5825 (0.0413) | 0.5472 (0.0723) | 0.6212 (0.0531) |
| 0.2 wt% 2'-FL + 0.1 wt% 3'-GL | 0.4290 (0.0280)* | 0.4822 (0.0451)* | 0.4647 (0.614)* |
| Observed effect | | | |
| 0.2 wt% 2'-FL + 0.1 wt% 3'-GL | 0.5712 (0.062) | 0.687 (90.090) | 0.6822 (0.123) |
| Calculated effect | | | |

| | | | |
|---|---|---|---|
| * paired one-tailed (Wilcoxon) t test p<0.05 when compared with the 2'-FL + 3'-GL calculated effect. | | | |

T cell stimulation of the human PBMCs resulted in the significant increase of IL-4 and IL-13. Pre-incubation of the cells with 2'-FL had no effect on the levels of IL-4 and IL-13 as compared to controls. However, a decrease was detected in the presence of 3'-GL as compared to control. Moreover, the combination of 2'-FL and 3'-GL induced significantly lower levels of IL-4 and IL-13 as compared to control and 2'-FL. Interestingly, these reduced IL-4 and IL-13 levels were significantly lower than could be expected based on calculations of the individual effects of 2'-FL and 3'-GL, see Table 9.

These data show an unexpected reduced Th2 type of responsiveness upon T cell stimulation within the total PBMC population when the cells are in the presence of a combination of 2'-FL and 3'-GL, compared to 3'-GL or 2'FL alone.

The cytokines regulate cellular responses on transcriptional level, while chemokines play a role in recruiting inflammatory cells to the sites on inflammation. The chemokine CCL17 (thymus and activation-regulated chemokine) is a potent chemoattractant for Th2 lymphocytes and is thought to play an important role in inflammatory diseases like allergy. In example, serum CCL17 levels sharply reflect the disease activity of atopic dermatitis, which is considered to be a Th2-dominant inflammatory skin disease, especially in the acute phase.

Human T cell stimulation resulted in significant increase in CCL17, see Table 9. Although pre-incubation with either 2'-FL or 3'-GL had no significant effect on CCL17 levels within T cell stimulated PBMCs, a significant decrease was detected in the levels of CCL17 when the activated PBMCs were preincubated with both 2'-FL and 3'-GL as compared to single 2'-FL and 3'-GL. Based on changes induced by individual components as compared to control levels, one can calculate the expected change when combining the interventions. Interestingly when T cell stimulated PBMCs were cultured in the presence of the combination of 2'-FL and 3'-GL lower CCL17 levels were induced than expected. The changes in CCL17 levels indicate an unexpected further reduction of Th2 type responsiveness upon T cell stimulation within the total PBMC population when the cells are in the presence of a combination of 2'-FL and 3'-GL. These CCL17 data are in line with the IL-4 and IL-13 data.

The total pool of isolated human PBMCs is a diverse pool of immune cells, which can respond directly and differently to provided HMOs. Although cells become more metabolically active, Th2 mediators IL-4, IL-13 and CCL17 levels were not significantly affected by single 2'-FL exposure, while single 3'-GL exposure resulted in a reduction of these mediator levels. Interestingly, the simultaneous exposure of 2'-FL and 3'-GL statistically significantly reduced IL-4, IL-13 and CCL17 levels, thereby reducing the Th2 type of responses. These data indicate that the addition of 3'-GL to 2'-FL has the potential to reduce allergy development.

### Example 6: 2'-FL increases the butyrate formation by the microbiota, in particular if also GOS is present

A faecal sample from a 3 months old healthy infant born via C-section, exclusively breastfed with no history of antibiotic usage, was used as inoculum to simulate the infant intestinal microbiota in the colon compartments of a quad-SHIME^{®}- a dynamic model of the human gastrointestinal tract comprises 4 SHIME^{®} units running in parallel and each SHIME^{®} unit is composed of 3 reactors simulating the stomach and small intestine, proximal and distal colons.

SCFA profiles showed that acetate is the most abundant in the distal colon, followed by propionate (Table 10). The concentrations of acetate and propionate were higher in the presence of scGOS/IcFOS and scGOS/IcFOS/2'-FL than in the control and 2'-FL-supplemented units. Similar observations were also seen in the proximal colons (data not shown). Interestingly, butyrate was generated earlier in the distal colon and at a higher concentration in the presence of 2'-FL and scGOS/IcFOS/2'-FL relative to the control and the scGOS/IcFOS groups. The level of iso-butyrate, a branched SCFA resulting from the proteolytic fermentation, was reduced in the distal colon in the presence of scGOS/IcFOS/2'-FL and scGOS/IcFOS.

**Table 10: Short-chain fatty acids produced in the distal colons of the un-supplemented (control) and supplemented SHIME^{®} units at D1 to Day 3 (D1 - D3), Day 4 to Day 11 (D4 - D11), and Day 12 to D15 (D12 - D15).**

| | **Control** | | | **scG/lcF** | | | **2'-FL** | | | **2'-FL + scG/lcF** | | |
|---|---|---|---|---|---|---|---|---|---|---|---|---|
| | **D1-D3** | **D4-D11** | **D12-D15** | **D1-D3** | **D4-D11** | **D12-D15** | **D1-D3** | **D4-D11** | **D12-D15** | **D1-D3** | **D4-D11** | **D12-D15** |
| **Acetate (Mean ± SD)** | 37 ± 3.77 | 27.77 ± 2.32 | 27.31 ± 5.43 | 58.83 ± 17.67 | 84.3 ± 9.8 | 82.13 ± 3.35 | 38.67 ± 10.25 | 26.22 ± 0.97 | 22.96 ± 1.04 | 58.5 ± 9.26 | 83.94 ± 6.8 | 84.43 ± 9.20 |
| **Propionate (Mean ± SD)** | 10 ± 1 | 6.58 ± 0.58 | 6.5 ± 1.08 | 13.83 ± 3.4 | 18.75 ± 3.5 | 18.63 ± 1.03 | 10.17 ± 3.01 | 6.58 ± 0.49 | 6.13 ± 0.63 | 14.17 ± 1.04 | 20.17 ± 2.36 | 21.50 ± 2.55 |
| **Butyrate (Mean ± SD)** | 0 ± 0 | 0.05 ± 0.12 | 0.71 ± 0.15 | 0 ± 0 | 0.04 ± 0.1 | 0.29 ± 0.08 | 0 ± 0 | 0.74 ± 0.49 | 0.99 ± 0.09 | 0 ± 0 | 0.64 ± 0.32 | 1.06 ± 0.15 |
| **Iso-Butyrate (Mean ± SD)** | 0 ± 0 | 0.6 ± 0.93 | 1.29 ± 0.28 | 0 ± 0 | 0.43 ± 0.49 | 0.20 ± 0.23 | 0 ± 0 | 0.68 ± 0.75 | 0.93 ± 0.21 | 0 ± 0 | 0.16 ± 0.19 | 0 ± 0 |

The glycoprofile data revealed that 2'-FL was not metabolized when supplemented alone, but only utilised in the presence of scGOS/IcFOS where it was slowly metabolised across the proximal and distal colon. All other carbohydrates including scGOS were depleted within the first hour in the proximal colon. It was shown that 2'-FL was only fermented in the presence of other GOS, in particular GOS/IcFOS resulting in a microbial eco-system that is suggested to confer health benefits.
scGOS/IcFOS/2'-FL enhanced the production of butyrate, an important SCFA for the gut barrier function. scGOS/IcFOS/2'-FL resulted in a surprisingly lower level of iso-butyrate, which is an indication of a less proteolytic activity in the colon.

### Example 7: inhibition of pathogens in the microbiota by 2'-FL, 3'-GL and butyrate

Anaerobic fermentation of fecal slurry samples was tested in a BioLector Pro microfluidics mini multifermentor. Fecal slurry samples were collected from breast fed infants and from formula fed infants. These fecal slurry samples were processed by adding 0.6 grams feces in 40 ml Baby Reichardt V.6 medium + mucus + Ammonium sulfate + lactate and acetate. The resulting solutions were inserted to the BioLector Pro microfluidics mini multifermentor. The test legs were supplemented with 3-GL, 2'-FL, 3-GL + 2'-FL, and GOS/FOS. The control leg was supplemented with sterile water.

In addition the test legs were supplemented with *Clostridium difficile* C153 (difficile agar), *Salmonella enteriditis* S29 (XLD agar), *Cronobacter sakasakii* E71 (chromogenic agar) or *Klebsiella pneumonia* K2 (Simons citrate inositol agar). For every NDO and for the control also a pathogen free culture was prepared.

After fermentation the fermented solutions were tested for SCFA content (in particular acetic acid, propionic acid, butyric acid and isobutyric acid), ammonia content, lactate content and pathogen concentration. Also DNA-isolation + identification and 16s sequencing was performed to determine the composition of the microbiota.

A 32 well plate that can handle low pH was used. The wells of this plate were filled with fecal solution. 2.5% (w/v) of the different sterile carbohydrate solutions (3'-GL, 2'-FL, 2'-FL/3'-GL (2.0+0.5%). and Glucose were added to the fecal slurry according to a template.

The experiment was started, and pH setpoint was either 5.5 (facial inoculum from infant 1, vaginally born, breast fed, 5 months of age) or pH 6.0 (Inoculum from infant 2, vaginally born, breast fed, 5 months of age) with continuous pH regulation, and temperature 37°C, humidity 85%, OD control. At 4, 8 and 24 hours a sample is taken for CFU determination on TOS-propionate MUP agar (total Bifidobacteria), XLD agar for *Salmonella enteriditis* S29, and Simons citrate inositol agar for *Klebsiella pneumonia* K2 and for SCFA, D- and L Lactate and ammonia analysis. Fecal pellet was used for 16s DNA sequencing. For both inocula the extent of fermentation, as measured by NaOH consumption, i.e. acid production, was highest with the combination of 3'-GL/2'-FL, when compared to 3'-GL or 2'-FL alone. The rate of initial acidification was high for 3'-GL and for 3'-GL/2'-FL. In general 2'-FL alone resulted in slower and lower acidification. As the amount of carbohydrates that can be fermented is the same in the reaction vessels, the higher total acidification with the combination is indicative for an unexpected, synergistic effect of the combination of 2'-FL and 3'-GL. The SCFA that were produced was for the largest part acetic acid. Also L-lactic acid was produced.

Growth of bifidobacteria was observed with 3'-GL, 2'-FL and with the mixture 2'-FL/3'-GL and growth stimulation was in general very similar. However, at 24 h the highest level was observed with the 3'-GL/2'-FL mixture for baby 1. Growth of Enterobacteriaceae was also observed, and was very similar under the conditions tested, but was lowest at 8 h for the combination of 2'-FL/3'-GL for the inoculum of baby 1. 16s Microbiota sequencing data at this time point showed a relative decrease was seen of the phylum of Proteobacteria (main contributor being the genus Escherichia). At the end of fermentation, when carbohydrates were depleted the 2'-FL/3'-GL fed microbiota was able to retain a more positive microbiota composition than the controls (glucose and blanc). For the inoculum of baby 2 the effect on bifidobacteria was highest in the presence of 3'-GL, and the growth reducing effect on enterobacteriacea was best when a combination of 3'-GL/2'-FL was used.

Under conditions where the vessels were spiked with the mixture of pathogens, in general a slightly reduced acidification was observed when compared to the conditions where there was no spiking with pathogens. However, the effects of 2'-FL, 3'-GL and 2'-FL/3'-GL on acidification, as determined by NaOH consumption, was not affected, and again was highest with 3'-GL/2'-FL for both inocula. For the inoculum of baby 1 Salmonella growth was most restricted by 2'-FL, whereas Klebsiella growth was most inhibited by the combination of 3'-GL/2-'FL. For the inoculum of baby 2 Salmonella growth was most restricted by 2'-FL, whereas Klebsiella growth was most inhibited by 3'-GL or the combination of 3'-GL/2'-FL. For both inocula the outgrowth of C difficile was restricted under all the conditions

These results are indicative for an improved effect on the intestinal microbiota function and composition combination of 2'-FL and 3'-GL going beyond the effects of 2'-FL alone or 3'-GL alone. This indicates that a composition with dietary butyrate and 2'FL will have a further improved effect on the microbiota when further 3'GL is present.

### Example 8: Infant formula

Infant formula, intended for infants of 0 to 6 months of age, comprising per 100 ml, after reconstituting 13.7 g powder to an end volume of 100 ml:
- 66 kcal,
- 1.3 g protein (whey protein/casein wt ratio 1/1),
- 7.3 g digestible carbohydrates (mainly being lactose),
- 3.4 gram fat (of which about 50 wt.% bovine milk fat, the remainder being vegetable oils, fish oil and microbial oil). Based on total fatty acids the amount of butyric acid is 1.48 wt.% , the amount of arachidonic acid is 0.52 wt.%, the amount of eicosapentaenoic acid is 0.11 wt.%, the amount of docosahexaenoic acid is 0.52 wt.%,
- 0.9 g non-digestible oligosaccharides, of which 0.1 g 2'-FL (source Jennewein), 0.08 g long chain fructo-oligosaccharides (source RaftilineHP), 0.72 g galacto-oligosaccharides (of which about 25 mg 3'galactosyllactose obtained by fermentation, the remainder being galacto-oligosaccharides from Vivinal GOS),
- Minerals, vitamins, trace elements and other micronutrients as according to directives for infant formula,
- Part of the formula about 26 wt.% based on dry weight, is derived from the Lactofidus product fermented by S. thermophilus and B. breve strains, resulting in about (about 0.28 wt.% lactic acid based on dry weight of the composition, of which more than 95 wt.% is in the L-form.

### Example 9: Follow on formula

Follow on formula, intended for infants over 6 months of age, comprising per 100 ml, after reconstituting 14.55 g powder to an end volume of 100 ml:
- 68 kcal,
- 1.36 g protein (whey protein/casein wt ratio 4/6),
- 8.1 g digestible carbohydrates (mainly being lactose),
- 3.2 gram fat (of which about 50 wt.% bovine milk fat, the remainder being vegetable oils, fish oil and microbial oil). Based on total fatty acids the amount of butyric acid is 1.47 wt.% , the amount of arachidonic acid is 0.29 wt.%, the amount of eicosapentaenoic acid is 0.12 wt.%, the amount of docosahexaenoic acid is 0.56 wt.%,
- 0.85 g non-digestible oligosaccharides, of which 0.05 g 2'-FL (source Jennewein, name?), 0.08 g long chain fructo-oligosaccharides (source RaftilineHP), 0.72 g galacto-oligosaccharides (of which about 25 mg 3'galactosyllactose obtained by fermentation, the remainder being galacto-oligosaccharides from Vivinal GOS),
- Minerals, vitamins, trace elements and other micronutrients as according to directives for infant formula,
- Part of the formula, about 26 wt.% based on dry weight, is derived from the Lactofidus product fermented by S. thermophilus and B. breve strains, resulting in about 0.28 wt.% lactic acid based on dry weight of the composition, of which more than 95 wt.% is in the L-form.

### Example 10: Young child formula

Follow on formula, intended for young children over 12 months of age up to 36 months of age, comprising per 100 ml, after reconstituting 15.07 g powder to an end volume of 100 ml:
- 65 kcal,
- 1.3 g protein (whey protein/casein wt ratio 4/6),
- 8.7 g digestible carbohydrates (mainly being lactose),
- 2.6 gram fat (of which about 10 wt.% bovine milk fat, the remainder being vegetable oils, fish oil). Based on total fatty acids the amount of butyric acid is about 0.35 wt.%, the amount of eicosapentaenoic acid is 0.42 wt.%, the amount of docosahexaenoic acid is 0.63 wt.%,
- 1,22 g non-digestible oligosaccharides, of which 0.02 g 2'-FL (source Jennewein, name?), 0.12 g long chain fructo-oligosaccharides (source RaftilineHP), 1,08 g galacto-oligosaccharides (of which about 17 mg 3'galactosyllactose obtained by fermentation, the remainder being galacto-oligosaccharides from Vivinal GOS),
- Minerals, vitamins, trace elements and other micronutrients as according to directives for infant formula,
- Part of the formula, about 18 wt.% based on dry weight, is derived from the Lactofidus product fermented by S. thermophilus and B. breve strains, resulting in about (0.2 wt.% lactic acid based on dry weight of the composition, of which more than 95 wt.% is in the L-form.

## Claims

1. A nutritional composition for infants or young children, which is not human milk, comprising:
a. 2'fucosyllactose (2'-FL), and
b. dietary butyrate.

2. The nutritional composition according to claim 1 further comprising beta 3'galactosyllactose (beta3'-GL).

3. The nutritional composition according to any one of the preceding claims, wherein the composition is at least partly fermented by lactic acid producing bacteria and comprises 0.1 to 1.5 wt.% of the sum of lactic acid and lactate based on dry weight of the nutritional composition, and wherein at least 90 wt.% of the sum of lactic acid and lactate is L-lactic acid and L-lactate.

4. The nutritional composition according to any one of the preceding claims, wherein the composition further comprises LC-PUFA selected form the group of DHA, ARA, and EPA, preferably DHA and EPA, preferably DHA, EPA and ARA, wherein the nutritional composition preferably comprises at least 1 wt.% of the sum of DHA, ARA and EPA based on total fatty acids.

5. The nutritional composition according to any one of the preceding claims, wherein the formula further comprises galacto-oligosaccharides and/or fructo-oligosaccharides.

6. The nutritional composition according to any one of the preceding claims, wherein the nutritional composition is selected from the group consisting of infant formula, a follow-on formula or a young child formula, preferably an infant formula.

7. The nutritional composition according to any one of the preceding claims, wherein the composition comprises (i) 0.02 to 1 gr 2'-FL per 100 ml nutritional composition; (ii) 0.15 to 7.5 wt.% based on dry weight; and/or (iii) 0.03 to 1.5 g per 100 kcal.

8. The nutritional composition according to any one of the preceding claims, wherein the nutritional composition comprises (i) 0.010 to 0.250 g beta3'-GL per 100 ml; (ii) 0.075 to 2 wt.% based on dry weight; and/or (iii) 0.015 to 0.4 g per 100 kcal.

9. The nutritional composition according to any one of the preceding claims, wherein the nutritional composition comprises (i) 0.3 to 5 wt.% dietary butyrate based on total fatty acids; (ii) 10 mg to 175 mg per 100 ml; (iii) 15 to 250 mg per 100 kcal; and/or (iv) 0.075 to 1.3 wt.% based on dry weight of the nutritional composition.

10. The nutritional composition according to any one of the preceding claims, wherein the nutritional composition comprises (i) 0.2 to 5 g of the sum of galacto-oligosaccharides and fructo-oligosaccharides per 100 ml; and/or (ii) 0.3 to 7.5 g per 100 kcal, 1.5 to 35 wt.% based on dry weight.

11. The nutritional composition according to any of the preceding claims, for use in a method for the treatment or the prevention of infections, in particular intestinal infections.

12. The nutritional composition according to any of the preceding claims, for use in a method of inducing oral tolerance to allergens.

13. Nutritional composition for use according to claim 11 or 12, wherein the nutritional composition is administered to infants or young children, preferably infants.

## Patentansprüche

1. Eine Nahrungsmittel- bzw. Nährstoffzusammensetzung bzw. eine Zusammensetzung zu Ernährungszwecken für Säuglinge oder Kleinkinder, die keine menschliche Milch ist, die Folgendes aufweist:
a. 2'-Fucosyllactose (2'-FL), und
b. diätetisches Butyrat.

2. Die Nahrungsmittelzusammensetzung nach Anspruch 1, die ferner Beta-3'-Galactosyllactose (Beta-3'-GL) aufweist.

3. Die Nahrungsmittelzusammensetzung nach einem der vorhergehenden Ansprüche, wobei die Zusammensetzung zumindest teilweise mittels Milchsäure produzierender Bakterien fermentiert ist und die 0,1 bis 1,5 Gew.-% der Summe von Milchsäure und Lactat aufweist, und zwar bezogen auf das Trockengewicht der Nahrungsmittelzusammensetzung, und wobei mindestens 90 Gew.-% der Summe von Milchsäure und Lactat L-Milchsäure und L-Lactat sind.

4. Die Nahrungsmittelzusammensetzung nach einem der vorhergehenden Ansprüche, wobei die Zusammensetzung ferner LC-PUFA aufweist, und zwar ausgewählt aus der Gruppe von DHA, ARA und EPA, vorzugsweise DHA und EPA, vorzugsweise DHA, EPA und ARA, wobei die Nahrungsmittelzusammensetzung vorzugsweise mindestens 1 Gew.-% der Summe von DHA, ARA und EPA bezogen auf die gesamten Fettsäuren aufweist.

5. Die Nahrungsmittelzusammensetzung nach einem der vorhergehenden Ansprüche, wobei die Formel ferner Galacto-Oligosaccharide und/oder Fructo-Oligosaccharide aufweist.

6. Die Nahrungsmittelzusammensetzung nach einem der vorhergehenden Ansprüche, wobei die Nahrungsmittelzusammensetzung ausgewählt ist aus der Gruppe bestehend aus Muttermilchersatz für Säuglinge, einem Muttermilchersatz als Folgenahrung oder einem Muttermilchersatz als Kleinkindernahrung, vorzugsweise einem Muttermilchersatz für Säuglinge.

7. Die Nahrungsmittelzusammensetzung nach einem der vorhergehenden Ansprüche, wobei die Zusammensetzung Folgendes aufweist:
(i) 0,02 bis 1 g 2'-FL pro 100 ml der Nahrungsmittelzusammensetzung;
(ii) 0,15 bis 7,5 Gew.-%, bezogen auf das Trockengewicht; und/oder
(iii) 0,03 bis 1,5 g pro 100 kcal.

8. Die Nahrungsmittelzusammensetzung nach einem der vorhergehenden Ansprüche, wobei die Nährstoffzusammensetzung Folgendes aufweist:
(i) 0,010 bis 0,250 g Beta-3'-GL pro 100 ml;
(ii) 0,075 bis 2 Gew.-%, bezogen auf das Trockengewicht; und/oder
(iii) 0,015 bis 0,4 g pro 100 kcal.

9. Die Nahrungsmittelzusammensetzung nach einem der vorhergehenden Ansprüche, wobei die Nahrungsmittelzusammensetzung Folgendes aufweist:
(i) 0,3 bis 5 Gew.-% diätetisches Butyrat, und zwar bezogen auf die gesamten Fettsäuren;
(ii) 10 mg bis 175 mg pro 100 ml;
(iii) 15 bis 250 mg pro 100 kcal; und/oder
(iv) 0,075 bis 1,3 Gew.-%, bezogen auf das Trockengewicht der Nahrungsmittelzusammensetzung.

10. Die Nahrungsmittelzusammensetzung nach einem der vorhergehenden Ansprüche, wobei die Nahrungsmittelzusammensetzung Folgendes aufweist:
(i) 0,2 bis 5 g der Summe von Galacto-Oligosacchariden und Fructo-Oligosacchariden pro 100 ml; und/oder
(ii) 0,3 bis 7,5 g pro 100 kcal, 1,5 bis 35 Gew.-%, bezogen auf das Trockengewicht.

11. Die Nahrungsmittelzusammensetzung nach einem der vorhergehenden Ansprüche zur Verwendung bei einem Behandlungsverfahren oder dem Vorbeugen von Infektionen, insbesondere von Darminfektionen.

12. Die Nahrungsmittelzusammensetzung nach einem der vorangehenden Ansprüche zur Verwendung bei einem Verfahren zum Induzieren von oraler Toleranz gegenüber Allergenen.

13. Die Nahrungsmittelzusammensetzung zur Verwendung nach Anspruch 11 oder 12, wobei die Nahrungsmittelzusammensetzung Säuglingen oder Kleinkindern, vorzugsweise Säuglingen, verabreicht wird.

## Revendications

1. Composition nutritionnelle pour nourrissons ou jeunes enfants, qui n'est pas du lait maternel humain, comprenant :
a. du 2'fucosyllactose (2'-FL), et
b. du butyrate alimentaire.

2. Composition nutritionnelle selon la revendication 1, comprenant en outre du bêta 3'galactosyllactose (bêta 3'-GL).

3. Composition nutritionnelle selon l'une quelconque des revendications précédentes, dans laquelle la composition est au moins partiellement fermentée par des bactéries productrices d'acide lactique et comprend de 0,1 à 1,5 % en poids de la somme d'acide lactique et de lactate sur la base du poids sec de la composition nutritionnelle, et dans laquelle au moins 90 % en poids de la somme de l'acide lactique et du lactate sont de l'acide L-lactique et du L-lactate.

4. Composition nutritionnelle selon l'une quelconque des revendications précédentes, dans laquelle la composition comprend en outre des LC-PUFA choisis dans le groupe comprenant DHA, ARA et EPA, de préférence DHA et EPA, de préférence DHA, EPA et ARA, dans laquelle la composition nutritionnelle comprend de préférence au moins 1 % en poids de la somme de DHA, ARA et EPA sur la base des acides gras totaux.

5. Composition nutritionnelle selon l'une quelconque des revendications précédentes, dans laquelle la formule comprend en outre des galacto-oligosaccharides et/ou des fructo-oligosaccharides.

6. Composition nutritionnelle selon l'une quelconque des revendications précédentes, dans laquelle la composition nutritionnelle est choisie dans le groupe consistant en une préparation pour nourrisson, une préparation de suite ou une préparation pour jeune enfant, de préférence une préparation pour nourrisson.

7. Composition nutritionnelle selon l'une quelconque des revendications précédentes, dans laquelle la composition comprend (i) de 0,02 à 1 g de 2'-FL pour 100 ml de composition nutritionnelle ; (ii) de 0,15 à 7,5 % en poids sur la base du poids sec ; et/ou (iii) de 0,03 à 1,5 g pour 100 kcal.

8. Composition nutritionnelle selon l'une quelconque des revendications précédentes, dans laquelle la composition nutritionnelle comprend (i) de 0,010 à 0,250 g de bêta 3'-GL pour 100 ml ; (ii) de 0,075 à 2 % en poids sur la base du poids sec ; et/ou (iii) de 0,015 à 0,4 g pour 100 kcal.

9. Composition nutritionnelle selon l'une quelconque des revendications précédentes, dans laquelle la composition nutritionnelle comprend (i) de 0,3 à 5 % en poids de butyrate alimentaire sur la base des acides gras totaux ; (ii) de 10 mg à 175 mg par 100 ml ; (iii) de 15 à 250 mg par 100 kcal ; et/ou (iv) de 0,075 à 1,3 % en poids sur la base du poids sec de la composition nutritionnelle.

10. Composition nutritionnelle selon l'une quelconque des revendications précédentes, dans laquelle la composition nutritionnelle comprend (i) de 0,2 à 5 g de la somme des galacto-oligosaccharides et des fructo-oligosaccharides pour 100 ml ; et/ou (ii) de 0,3 à 7,5 g pour 100 kcal, de 1,5 à 35 % en poids sur la base du poids sec.

11. Composition nutritionnelle selon l'une quelconque des revendications précédentes, pour une utilisation dans un procédé pour le traitement ou la prévention d'infections, en particulier d'infections intestinales.

12. Composition nutritionnelle selon l'une quelconque des revendications précédentes, pour une utilisation dans un procédé pour induire une tolérance orale aux allergènes.

13. Composition nutritionnelle pour une utilisation selon la revendication 11 ou 12, dans laquelle la composition nutritionnelle est administrée à des nourrissons ou de jeunes enfants, de préférence des nourrissons.
